(19) **Europäisches Patentamt / European Patent Office / Office européen des brevets**

(11) **EP 2 205 731 B1**

(12) **EUROPEAN PATENT SPECIFICATION**

(45) Date of publication and mention
of the grant of the patent:
**05.12.2012 Bulletin 2012/49**

(51) Int Cl.:
***C12N 9/54*** (2006.01)

(21) Application number: **08845861.7**

(22) Date of filing: **23.10.2008**

(86) International application number:
**PCT/US2008/080972**

(87) International publication number:
**WO 2009/058661 (07.05.2009 Gazette 2009/19)**

(54) **USE AND PRODUCTION OF CITRATE-STABLE NEUTRAL METALLOPROTEASES**

VERWENDUNG UND HERSTELLUNG CITRAT-STABILER NEUTRALER METALLOPROTEASEN

UTILISATION ET PRODUCTION DE MÉTALLOPROTÉASES NEUTRES STABLES VIS-À-VIS DES CITRATES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **31.10.2007 US 984046 P**

(43) Date of publication of application:
**14.07.2010 Bulletin 2010/28**

(73) Proprietor: **Danisco US Inc.**
**Palo Alto, CA 94304 (US)**

(72) Inventors:
• **HOMMES, Ronaldus, W. J.**
**Palo Alto, California 94304 (US)**
• **LIU, Amy**
**Palo Alto, California 94304 (US)**
• **SHAW, Andrew**
**Palo Alto, California 94304 (US)**
• **WALLACE, Louise**
**Palo Alto, California 94304 (US)**

(74) Representative: **Forrest, Graham Robert et al**
**Mewburn Ellis LLP**
**33 Gutter Lane**
**London**
**EC2V 8AS (GB)**

(56) References cited:
**EP-A- 0 357 157     WO-A-2007/044993**

• **IMANAKA T ET AL: "A NEW WAY ENHANCING THE THERMOSTABILITY OF PROTEASES" NATURE, NATURE PUBLISHING GROUP, LONDON, UK, vol. 324, no. 6098, 18 December 1986 (1986-12-18), pages 695-697, XP001026149 ISSN: 0028-0836**
• **KUBO M ET AL: "ALTERATION OF SPECIFIC ACTIVITY AND STABILITY OF THERMOSTABLE NEUTRAL PROTEASE BY SITE-DIRECTED MUTAGENESIS" APPLIED AND ENVIRONMENTAL MICROBIOLOGY, AMERICAN SOCIETY FOR MICROBIOLOGY, US, vol. 58, no. 11, 1 January 1992 (1992-01-01), pages 3779-3783, XP000914629 ISSN: 0099-2240**
• **HONJO M ET AL: "Cloning and expression of the gene for neutral protease of Bacillus amyloliquefaciens in Bacillus subtilis" JOURNAL OF BIOTECHNOLOGY, ELSEVIER SCIENCE PUBLISHERS, AMSTERDAM, NL, vol. 1, no. 5-6, 1 December 1984 (1984-12-01), pages 265-277, XP023944388 ISSN: 0168-1656 [retrieved on 1984-12-01]**

EP 2 205 731 B1

**Description**

**FIELD OF THE INVENTION**

The present invention provides methods and compositions comprising at least one neutral metalloprotease enzyme that has improved stability in the presence of a metal chelator. In some embodiments, the neutral metalloprotease finds use in cleaning and other applications comprising citrate, In some particularly preferred embodiments, the present invention provides methods and compositions comprising variant neutral metalloprotease(s) engineered to resist citrate-induced autolysis.

**BACKGROUND OF THE INVENTION**

Members of the genus *Bacillus* are Gram-positive bacteria that secrete a number of industrially useful enzymes, which can be produced cheaply in high volume by fermentation. Examples of secreted *Bacillus* enzymes are the subtilisin serine proteases, zinc containing neutral proteases, alpha-amylases, and cellulases. *Bacillus* proteases are widely used in the textile, laundry and household industries (Galante. Current Organic Chemistry. 7:1399-1422, 2003; and Showell, Handbook of Detergents, Part D: Formulation. Hubbard (ed.). NY: Taylor and Francis Group, 2006). Highly efficient color and stain removal from laundry require proteases. However, liquid preparations of cleaning and washing reagents typically contain builders, surfactants, and metal chelators, which have a destabilizing effect on most proteases.

In general, denaturants induce rapid protease degradation by autolysis. Thus, an understanding of the molecular mechanism of protease autolysis has been investigated as a means to create stable proteases (Eijsink et al., J Biotechnol, 113: 105-120, 2004). The elucidation of the autolytic pathway is complicated by (i) the high efficacy by which the protease degrades locally denatured protein states, and (ii) because multiple unfolded states are most likely present and distributed throughout the protein molecule, resulting in multiple and parallel autolysis pathways. The molecular mechanism of autolysis of thermolysin-like metalloproteases has been reported (Eijsink et al., Nat Struct Biol, 2:374-379. 1995; van den Burg et al., Biotechnol Appl Bioeng, 30:35-40. 1999; and Vriend. J Comput Aided Mol Des. 7:367-396. 1993). WO 2007/044993 describes the production and use of *Bacillus* neutral metalloprotease variants having improved storage stability.

There remains a need in the art however, for the elucidation of the mechanism of autolysis of additional industrially relevant metalloproteases. In particular, there remains a need for an understanding of citrate-induced autolysis of *Bacillus amyloliquefaciens* neutral protease (NprE), that can be used to design NprE variants having improved stability in the presence of a calcium chelator. This is especially important in the case of *B. amyloliquefaciens* NprE since this enzyme is dependent upon calcium for its structure and function, making it all the more susceptible to calcium-depleting agents such as citrate.

**SUMMARY OF THE INVENTION**

The present invention provides methods and compositions comprising at least one neutral metalloprotease enzyme as defined in the claims, that has improved stability in the presence of a metal chelator. In some embodiments, the neutral metalloprotease finds use in cleaning and other applications comprising citrate. In some particularly preferred embodiments, the present invention provides methods and compositions comprising variant neutral metalloprotease(s) engineered to resist citrate-induced autolysis.

The present invention provides isolated neutral metalloprotease variants having improved resistance to citrate-induced autolysis. The neutral metalloprotease variant is a *Bacillus* neutral metalloprotease variant having multiple mutations selected from M138L/V190I/D220P, and S129I/F130L/M138L/V190I/D220P. In preferred embodiments, the *Bacillus* is *B. amyloliquefaciens.* The neutral metalloprotease has at least about 45%, at least about 50%, at least about 53%, at least about 55%, at least about 57%, at least about 60%, at least about 63%, at least about 65%, at least about 67%, at least about 70%, at least about 73%, at least about 75%, at least about 77%, at least about 80%, at least about 85%, at least about 90%, at least about 95%, at least about 96%, at least about 97%, at least about 98%, or at least about 99% amino acid identity with the neutral metalloprotease comprising the amino acid sequence set forth as SEQ ID NO:3. The present invention also provides isolated nucleic acids encoding the neutral metalloprotease variants described herein, as well as expression vectors comprising the nucleic acids. Moreover, the present invention provides host cells comprising the expression vectors. In still further embodiments, the present invention provides neutral metalloprotease variants obtained from the host cells comprising the expression vectors.

In addition, the present invention provides isolated *B. amyloliquefaciens* neutral metalloprotease variants as defined in the claims having improved resistance to citrate-induced autolysis. In some preferred embodiments, the neutral metalloprotease variant has an amino acid sequence comprising substitutions at three, four or five positions selected from the group equivalent to positions 129. 130, 138. 190, and 220 of the amino acid sequence set forth as SEQ ID NO:

3. Also provided are isolated *B. amyloliquefaciens* neutral metalloprotease variants comprising multiple mutations of the amino acid sequence set forth as SEQ ID NO:3. wherein the multiple mutations are selected from M138L/V190I/D220P. and S129I/F130L/M138L/V190I/D220P. In some preferred embodiments, the isolated *B. amyloliquefaciens* neutral metalloprotease variants comprise the amino acid sequence set forth as SEQ ID NO: 19 (M138L/V190I/D220P), or SEQ ID NO:20 (S 129I/F130L/M138L/V 190I/D220P). The present invention also provides isolated nucleic acids encoding the neutral metalloprotease variants as defined in the claims, as well as expression vectors comprising nucleic acids encoding the neutral metalloprotease variants. Moreover, the present invention provides host cells comprising the expression vectors. In still further embodiments, the present invention provides neutral metalloprotease variants obtained from the host cells comprising the expression vectors.

Moreover, the present invention provides methods for producing an enzyme having neutral metalloprotease activity, comprising: transforming a host cell with an expression vector comprising a nucleic acid encoding the neutral metalloprotease variants; and cultivating the transformed host cell under conditions suitable for the production of the neutral metalloprotease. Some embodiments of the present invention optionally further comprise the step of harvesting the produced neutral metalloprotease. In preferred embodiments, the host cell is a *Bacillus* species, e.g. *B. subtilis*.

The present invention also provides compositions comprising an isolated neutral metalloprotease variant as defined in the claims having improved resistance to citrate-induced autolysis. In some embodiments, the composition further comprises at least one calcium ion and/or at least one zinc ion. In additional embodiments, the composition further comprises citrate, In some preferred embodiments, the composition is a cleaning composition (*e.g.,* detergent). In some particularly preferred embodiments, the composition further comprises at least one additional enzyme or enzyme derivative selected from the group consisting of proteases, amylases, lipases, mannanases, pectinases, cutinases, oxidoreductases, hemicellulases, and cellulases. In some embodiments of the present invention, the composition comprises: at least about 0.0001 weight percent of the neutral metalloprotease variant; or from about 0.001 to about 0.5 weight percent of the neutral metalloprotease variant. Some compositions of the present invention further comprise at least one adjunct ingredient. In some particularly preferred embodiments, the composition further comprises a sufficient amount of a pH modifier to provide the composition with a neat pH of from about 3 to about 5. the composition being essentially free of materials that hydrolyze at a pH of from about pH 3 to about pH 5. In some embodiments, the materials that hydrolyze at a pH of from about pH 3 to about pH 5 comprise at least one surfactant. In some preferred embodiments, the surfactant is a sodium alkyl sulfate surfactant comprising an ethylene oxide moiety. In some embodiments, the composition is a liquid.

In addition, the present invention provides animal feed compositions comprising an isolated neutral metalloprotease variant as defined in the claims having improved resistance to citrate-induced autolysis. In further embodiments textile processing compositions are provided comprising an isolated neutral metalloprotease variant having improved resistance to citrate-induced autolysis. In still further embodiments leather processing compositions are provided comprising an isolated neutral metalloprotease variant having improved resistance to citrate-induced autolysis.

Moreover, the present invention provides methods of cleaning, comprising the step of contacting a surface and/or an article comprising a fabric (*e.g.*, material) with a cleaning composition comprising an isolated neutral metalloprotease variant having improved resistance to citrate-induced autolysis. In some embodiments, the methods may optionally further comprise the step of rinsing the surface and/or fabric after contacting the surface or material with the cleaning composition.

## BRIEF DESCRIPTION OF THE DRAWINGS

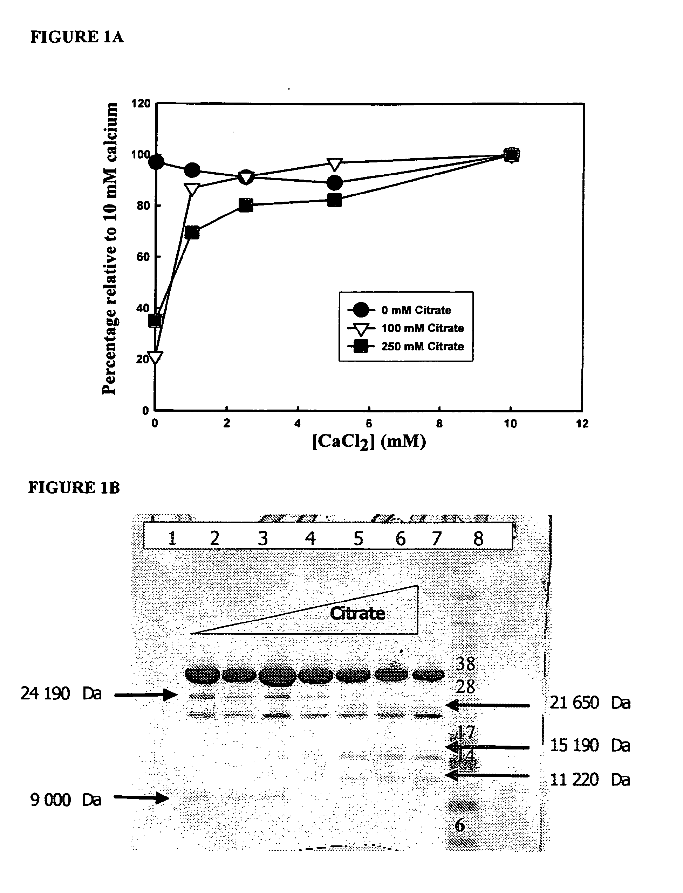

Figure 1 illustrates the citrate-induced autolysis of *Bacillus amyloliquefaciens* neutral protease (NprE). Panel A provides a graph showing inactivation of 0.4 mg/ml protease as function of citrate concentration (0-250 mM), and calcium concentration (0-10 mM) in 0.1 M Tris-HCl, pH 8.4. The remaining activity was measured using succinylated-casein/TNBSA and is relative to the activity measured in the presence of 10 mM calcium chloride. Panel B depicts a 10% SDS-PAGE analysis illustrating the autolysis pattern of protease induced by citrate. Approximately 0.4 mg/ml protein was incubated on ice for 100 minutes in 5 mM HEPES, pH 8.0. containing 0-100 mM citrate. The protease was inactivated with 0.1 N HCl to halt autolysis prior to gel loading. Lane I represents control protein incubated in the absence of citrate, lanes 2-7 contain protein in the presence of increasing citrate and lane 8 contains the molecular weight markers.

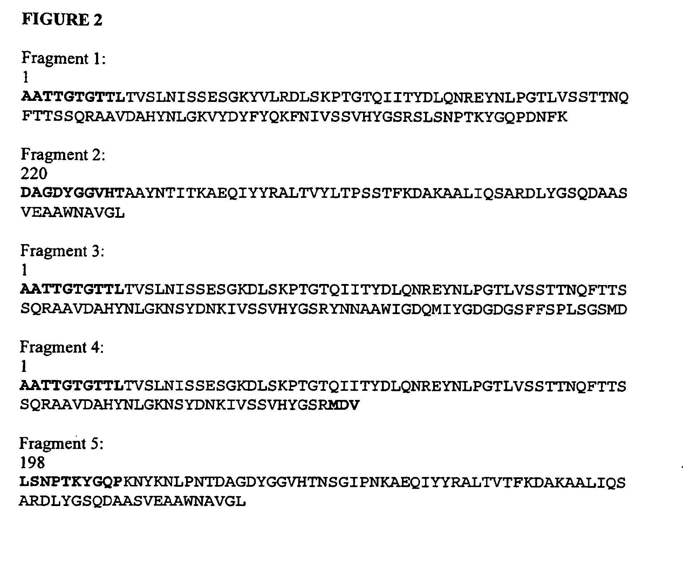

Figure 2 provides the amino acid sequence for the autolytic fragments of *B. amyloliquefaciens* neutral protease (NprE): fragment 1 (SEQ ID NO:13.), fragment 2 (SEQ ID NO:14); fragment 3 (SEQ ID NO:15); fragment 4 (SEQ ID NO:16); and fragment 5 (SEQ ID NO:17). The N-termini of the autolytic fragments are highlighted in bold. The numbering corresponds to the positions of the mature form of NprE.

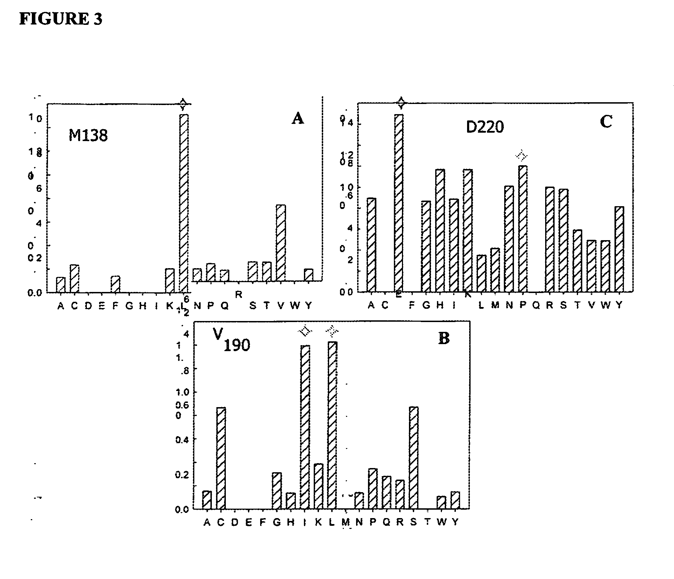

Figure 3 provides representative figures illustrating the activity data obtained by screening all possible amino acid replacements at three positions in NprE for citrate sensitivity. Panel A provides data from the activity screen of position M138. Panel B provides data from the activity screen of position D220. Panel C provides data from the activity screen of position V190. Screening was performed at 25 °C for 60 minutes in the absence and presence of 50 mM citrate in 25 mM HEPES, pH 8.0. The y-scale is arbitrary and represents the increase relative to the wild type protein.

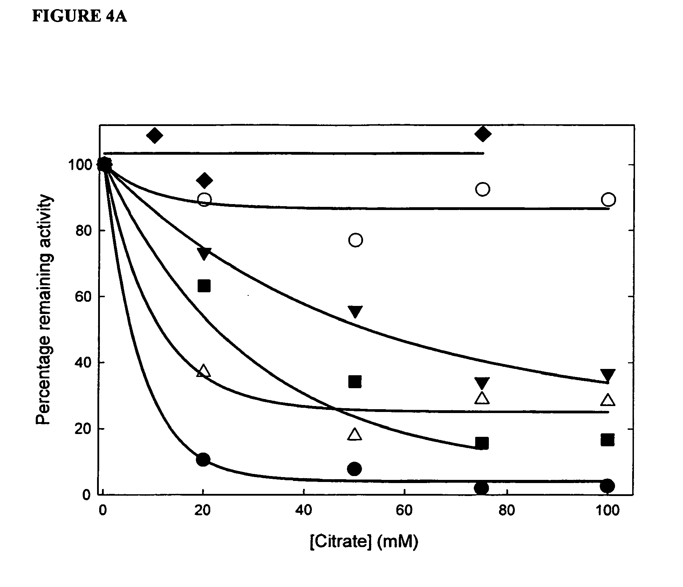

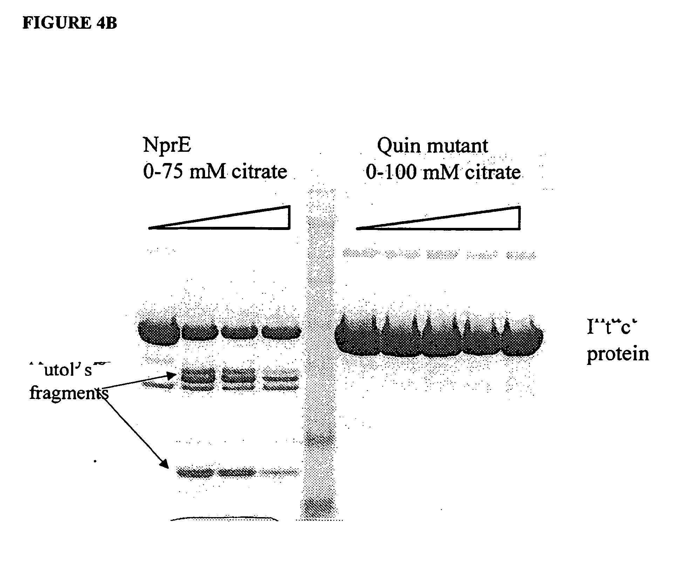

Figure 4 provides a graph showing activity of the neutral protease variants as a function of citrate concentration and incubation time. Panel A illustrates the citrate concentration dependence of activity measured after 60 minutes at room temperature for the wild-type protease (●), V190I (o), M138L (V), D220P (△), M138L-D220P (■) and S129I-F130L-M138L-V190I-D220P (♦). Panel B depicts a 10% SDS-PAGE analysis showing the effect of citrate on wild type NprE and the variant S129I-F130L-M138L-V190I-D220P. Approximately 0.4 mg/ml protein was incubated with increasing citrate concentration for 100 minutes at 4 °C. Lanes 1-4 illustrate the autolysis pattern for wild type as a function of citrate (0 - 75 mM), and lanes 6 - 10 show intact (non-autolysed) 5129I-F130L-M138L-V190I-D220P. Lanes 5 represents the standard molecular weight markers.

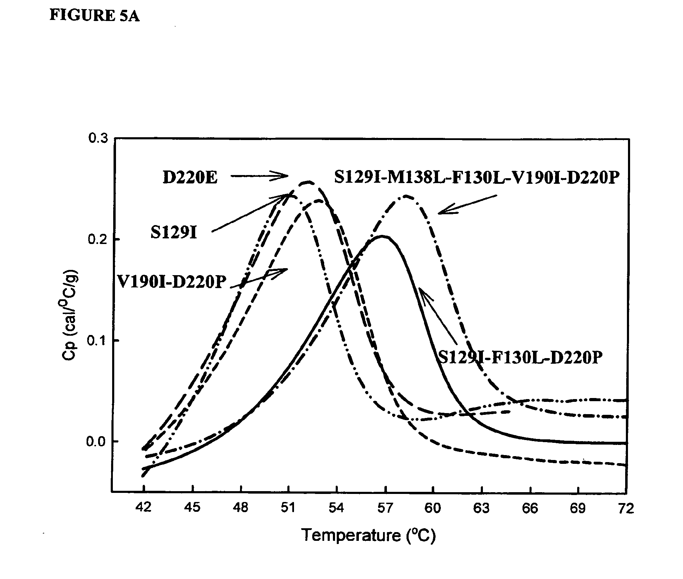

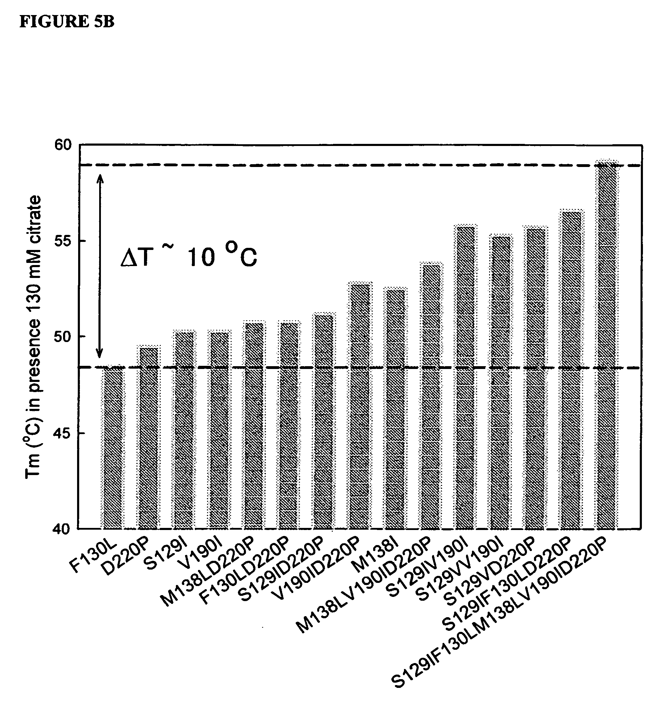

Figure 5 provides a graph showing the thermostability of wild type and variant proteases. Panel A provides representative calorimetric profiles for 0.4 mg/ml of protease variants S129I, D220E, V190I-D220P, S129I-F130L-D220P, and S129I-F130L-M138L-V190I-D220P in the presence of 130 mM citrate. Data was collected from 20-90 °C at a scan rate of 200 °C/hr using Auto-Cap VP-DSC (MicroCal, Northampton, MA, USA). All data shown is corrected for buffer baseline. The buffer was 5 mM HEPES, pH 8.0. Panel B provides a bar graph for a number of single, double and triple protease variants showing the incremental increase in the Thermal melting point (Tm's) in the presence of 130 mM citrate, pH 8.0.

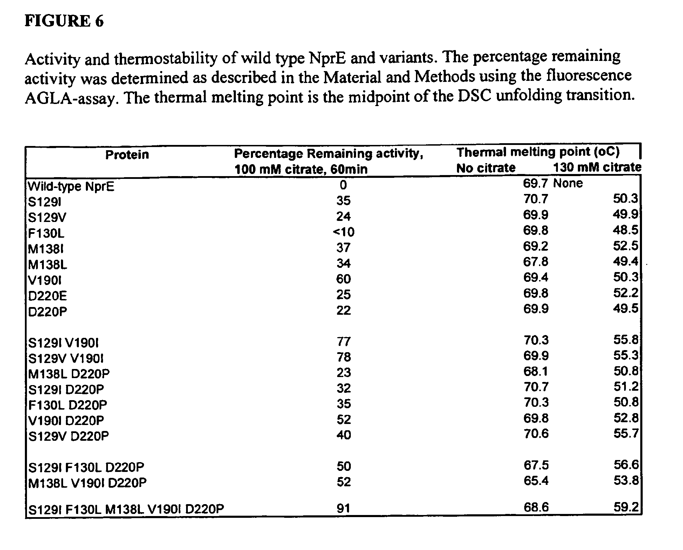

Figure 6 provides a table listing the activity and thermostability of wild type NprE and variants thereof.

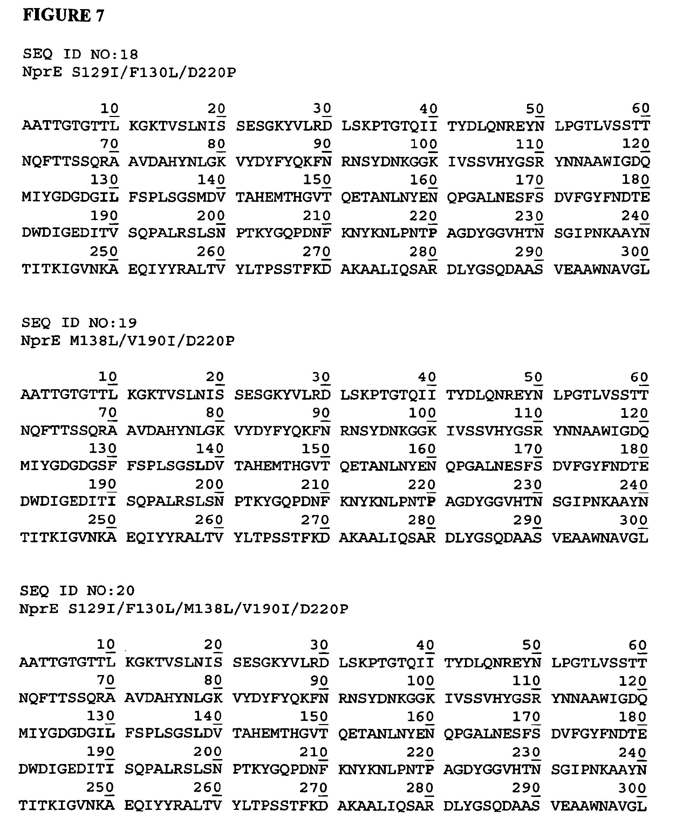

Figure 7 provides the amino acid sequences of exemplary citrate-stable NprE variants of the present invention. Panel A provides the amino acid sequence of the S129I-F130L-D220P NprE variant (SEQ ID NO: 18). Panel B provides the amino acid sequence of the M 138L-V 1901-D220P NprE variant (SEQ ID NO:19). Panel C provides the amino acid sequence of the S129IF 130L-M 138L-V190I-D220P NprE variant (SEQ ID NO:20).

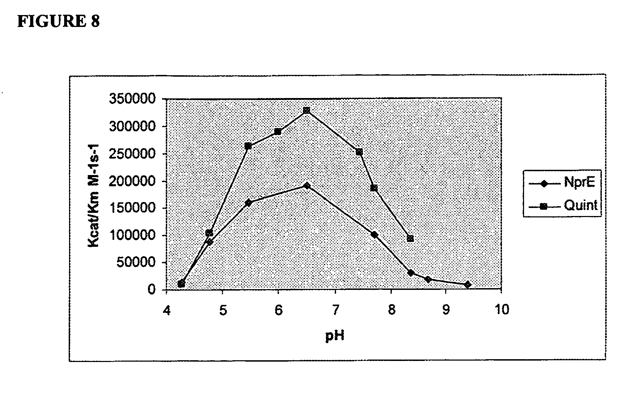

Figure 8 provides a graph of Kcat/Km versus pH. illustrating that both the wild type NprE and the quintuple NprE variant (S129I-F130L-M138L-V190I-D220P) have optimum activity towards an AGLA substrate at pH 6.5.

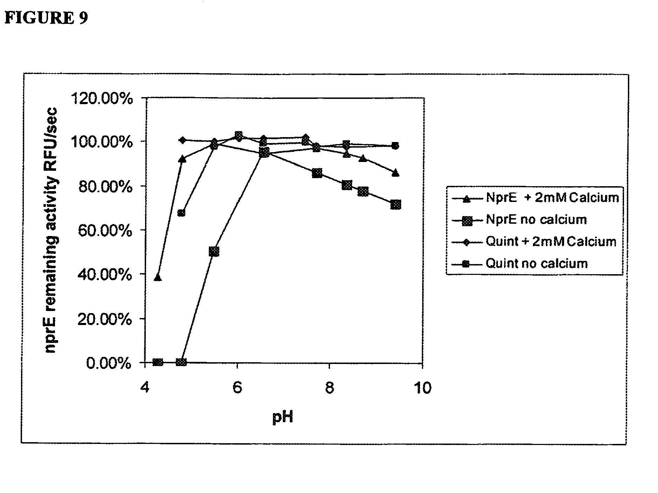

Figure 9 provides a graph of remaining NprE activity versus pH, illustrating that the addition of calcium stabilizes both the wild type NprE and the quintuple NprE variant (S129IF130L-M 138L-V 190I-D220P) at both low and high pH.

## GENERAL DESCRIPTION OF THE INVENTION

The present invention provides methods and compositions comprising at least one neutral metalloprotease enzyme as defined in the claims that has improved stability in the presence of a metal chelator. In some embodiments, the neutral metalloprotease finds use in cleaning and other applications optionally comprising citrate. In some particularly preferred embodiments, the present invention provides methods and compositions comprising variant neutral metalloprotease(s) as defined in the claims engineered to resist citrate-induced autolysis.

Unless otherwise indicated, the practice of the present invention involves conventional techniques commonly used in molecular biology, microbiology, and recombinant DNA, which are within the skill of the art. Such techniques are known to those of skill in the art and are described in numerous texts and reference works (*See e.g.,* Sambrook et al., "Molecular Cloning: A Laboratory Manual," Second Edition, Cold Spring Harbor, 1989; and Ausubel et al.. "Current Protocols in Molecular Biology," 1987).

Unless defined otherwise herein, all technical and scientific terms used herein have the same meaning as commonly understood by one of ordinary skill in the art to which this invention pertains. For example. Singleton and Sainsbury, Dictionary of Microbiology and Molecular Biology, 2d Ed.. John Wiley and Sons. NY (1994); and Hale and Marham, The Harper Collins Dictionary of Biology, Harper Perennial, NY (1991) provide those of skill in the art with a general dictionaries of many of the terms used in the invention. Although any methods and materials similar or equivalent to those described herein find use in the practice of the present invention, the preferred methods and materials are described herein. Accordingly, the terms defined immediately below are more fully described by reference to the Specification as a whole.

Also, as used herein, the singular "a", "an" and "the" includes the plural reference unless the context clearly indicates otherwise. Numeric ranges are inclusive of the numbers defining the range. Unless otherwise indicated, nucleic acids are written left to right in 5' to 3' orientation; amino acid sequences are written left to right in amino to carboxy orientation, respectively. It is to be understood that this invention is not limited to the particular methodology, protocols, and reagents described, as these may vary, depending upon the context they are used by those of skill in the art.

Furthermore, the headings provided herein are not limitations of the various aspects or embodiments of the invention, which can be had by reference to the specification as a whole. Accordingly, the terms defined immediately below are more fully defined by reference to the specification as a whole. Nonetheless, in order to facilitate understanding of the invention, a number of terms are defined below.

**Definitions**

Unless defined otherwise herein, all technical and scientific terms used herein have the same meaning as commonly understood by one of ordinary skill in the art to which this invention pertains. Although any methods and materials similar or equivalent to those described herein find use in the practice of the present invention, the preferred methods and materials are described herein. Accordingly, the terms defined immediately below are more fully described by reference to the Specification as a whole. Also, as used herein, the singular terms "a," "an," and "the" include the plural reference unless the context clearly indicates otherwise. Unless otherwise indicated, nucleic acids are written left to right in 5' to 3' orientation; amino acid sequences are written left to right in amino to carboxy orientation, respectively. It is to be understood that this invention is not limited to the particular methodology, protocols, and reagents described, as these may vary, depending upon the context they are used by those of skill in the art.

As used herein, the terms "protease." and "proteolytic activity" refer to a protein or peptide exhibiting the ability to hydrolyze peptides or substrates having peptide linkages. Many well known procedures exist for measuring proteolytic activity (Kalisz. "Microbial Proteinases." In: Fiechter (ed). Advances in Biochemical Engineering/Biotechnology, 1988). For example, proteolytic activity may be ascertained by comparative assays, which analyze the respective protease's ability to hydrolyze a commercial substrate. Exemplary substrates useful in such analysis of protease or proteolytic activity, include, but are not limited to di-methyl casein (Sigma C-9801), bovine collagen (Sigma C-9879), bovine elastin (Sigma E-1625), and bovine keratin (ICN Biomedical 90211I). Colorimetric assays utilizing these substrates are well known in the art (*See e.g.,* WO 99/34011; and U.S. Patent No. 6,376,450). The pNA assay (*See e.g.,* Del Mar et al., Anal Biochem, 99:316-320, 1979) also finds use in determining the active enzyme concentration for fractions collected during gradient elution. This assay measures the rate at which *p*-nitroaniline is released as the enzyme hydrolyzes the soluble synthetic substrate, succinyl-alanine-alanine-proline-phenylalanine-*p*-nitroanilide (sAAPF-*p*NA). The rate of production of yellow color from the hydrolysis reaction is measured at 410 nm on a spectrophotometer and is proportional to the active enzyme concentration. In addition, absorbance measurements at 280 nm can be used to determine the total protein concentration. The active enzyme/total-protein ratio gives the enzyme purity.

As used herein, the terms "NprE protease." and "NprE," refer to the neutral metalloproreases described herein. The NprE protease is the protease designated herein as purified MULTIFECT® Neutral or PMN obtained from *Bacillus amyloliquefaciens.* Thus, in some embodiments, the term "PMN protease" refers to a naturally occurring mature protease derived from *Bacillus amyloliquefaciens* having substantially identical amino acid sequences as provided in SEQ ID NO: 3. In alternative embodiments, the present invention provides portions of the NprE protease.

The term *"Bacillus* protease homologues" refers to naturally occurring proteases having substantially identical amino acid sequences to the mature protease derived from *Bacillus amyloliquefaciens* or polynucleotide sequences which encode for such naturally occurring proteases, and which proteases retain the functional characteristics of a neutral metalloprotease encoded by such nucleic acids.

As used herein, the terms "NprE variant." and "NprE protease variant." are used in reference to proteases that are similar to the wild-type NprE. particularly in their function, but have mutations in their amino acid sequence that make them different in sequence from the wild-type protease.

As used herein. *"Bacillus* ssp." refers to all of the species within the genus *"Bacillus,"* which are Gram-positive bacteria classified as members of the Family *Bacillaceae.* Order *Bacillales,* Class *Bacilli.* The genus *"Bacillus"* includes all species within the genus *"Bacillus,"* as known to those of skill in the art, including but not limited to *B. subtilis, B. licheniformis, B. lentus, B. brevis, B. stearothermophilus, B. alkalophilus, B. amyloliquefaciens, B. clausii, B. halodurans, B. megaterium, B. coagulans, B. circulans, B. lautus,* and *B. thuringiensis.* It is recognized that the genus *Bacillus* continues to undergo taxonomical reorganization. Thus, it is intended that the genus include species that have been reclassified, including but not limited to such organisms as *B. stearothermophilus,* which is now named *"Geobacillus stearothermophilus."* The production of resistant endospores in the presence of oxygen is considered the defining feature of the genus *Bacillus,* although this characteristic also applies to the recently named *Alicyclobacillus, Amphibacillus, Aneurinibacillus, Anoxybocillus, Brevibacillus, Filobacillus, Gracilibacillus, Halobacillus, Paenibacillus, Salibacillus. Thermobacillus, Ureibacillus,* and *Virgibacilll ̦s.*

Related (and derivative) proteins comprise "variant proteins." In some preferred embodiments, variant proteins differ from a parent protein and one another by a small number of amino acid residues. The number of differing amino acid residues may be one or more, preferably 1, 2, 3, 4, 5, 10, 15. 20, 30, 40, 50. or more amino acid residues. In some preferred embodiments, the number of different amino acids between variants is between 1 and 10. The variant proteins comprise at least 45%, 50%, 55%, 60%. 65%, 70%, 75%, 80%. 85%, 90%, 95%. 97%, 98%, or 99% amino acid sequence identity. Additionally, a related protein or a variant protein as used herein, refers to a protein that differs from another related protein or a parent protein in the number of prominent regions.

Several methods are known in the art that are suitable for generating variants of the enzymes of the present invention, including but not limited to site-saturation mutagenesis, scanning mutagenesis, insertional mutagenesis, random mutagenesis, site-directed mutagenesis, and directed-evolution, as well as various other recombinatorial approaches.

Characterization of wild-type and mutant proteins is accomplished via any means or "test" suitable and is preferably based on the assessment of properties of interest. For example, pH and/or temperature, as well as detergent and /or oxidative stability is/are determined in some embodiments of the present invention. Indeed, it is contemplated that enzymes having various degrees of stability in one or more of these characteristics (pH, temperature, proteolytic stability, detergent stability, and/or oxidative stability) will find use.

The terms "polynucleotide" and "nucleic acid", used interchangeably herein, refer to a polymeric form of nucleotides of any length, either ribonucleotides or deoxyribonucleotides. These terms include, but are not limited to, a single-, double- or triple-stranded DNA, genomic DNA, cDNA, RNA, DNA-RNA hybrid, or a polymer comprising purine and pyrimidine bases, or other natural, chemically, biochemically modified, non-natural or derivatized nucleotide bases. The following are non-limiting examples of polynucleotides: genes, gene fragments, chromosomal fragments, ESTs, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes, and primers. In some embodiments, polynucleotides comprise modified nucleotides, such as methylated nucleotides and nucleotide analogs, uracil, other sugars and linking groups such as fluororibose and thioate, and nucleotide branches. In alternative embodiments, the sequence of nucleotides is interrupted by non-nucleotide components.

As used herein, the terms "DNA construct" and "transforming DNA" are used interchangeably to refer to DNA used to introduce sequences into a host cell or organism. The DNA may be generated *in vitro* by PCR or any other suitable technique(s) known to those in the art. In particularly preferred embodiments, the DNA construct comprises a sequence of interest (*e.g.*, as an incoming sequence). In some embodiments, the sequence is operably linked to additional elements such as control elements (*e.g.*, promoters, etc.). The DNA construct may further comprise a selectable marker. It may further comprise an incoming sequence flanked by homology boxes. In a further embodiment, the transforming DNA comprises other non-homologous sequences, added to the ends (*e.g.*, stuffer sequences or flanks). In some embodiments, the ends of the incoming sequence are closed such that the transforming DNA forms a closed circle. The transforming sequences may be wild-type, mutant or modified. In some embodiments, the DNA construct comprises sequences homologous to the host cell chromosome. In other embodiments, the DNA construct comprises non-homologous sequences. Once the DNA construct is assembled *in vitro* it may be used to: 1) insert heterologous sequences into a desired target sequence of a host cell, and/or 2) mutagenize a region of the host cell chromosome (*i.e.*, replace an endogenous sequence with a heterologous sequence). 3) delete target genes; and/or introduce a replicating plasmid into the host.

As used herein, the terms "expression cassette" and "expression vector" refer to nucleic acid constructs generated recombinantly or synthetically, with a series of specified nucleic acid elements that permit transcription of a particular nucleic acid in a target cell. The recombinant expression cassette can be incorporated into a plasmid, chromosome, mitochondrial DNA, plastid DNA, virus, or nucleic acid fragment. Typically, the recombinant expression cassette portion of an expression vector includes, among other sequences, a nucleic acid sequence to be transcribed and a promoter. In preferred embodiments, expression vectors have the ability to incorporate and express heterologous DNA fragments in a host cell. Many prokaryotic and eukaryotic expression vectors are commercially available. Selection of appropriate expression vectors is within the knowledge of those of skill in the art. The term "expression cassette" is used interchangeably herein with "DNA construct," and their grammatical equivalents. Selection of appropriate expression vectors is within the knowledge of those of skill in the art.

As used herein, the term "vector" refers to a polynucleotide construct designed to introduce nucleic acids into one or more cell types. Vectors include cloning vectors, expression vectors, shuttle vectors, plasmids, cassettes and the like. In some embodiments, the polynucleotide construct comprises a DNA sequence encoding the protease (*e.g.*, precursor or mature protease) that is operably linked to a suitable prosequence (e.g., secretory, etc.) capable of effecting the expression of the DNA in a suitable host.

As used herein, the term "plasmid" refers to a circular double-stranded (ds) DNA construct used as a cloning vector, and which forms an extrachromosomal self-replicating genetic element in some eukaryotes or prokaryotes, or integrates into the host chromosome.

As used herein in the context of introducing a nucleic acid sequence into a cell, the term "introduced" refers to any method suitable for transferring the nucleic acid sequence into the cell. Such methods for introduction include but are not limited to protoplast fusion, transfection, transformation, conjugation, and transduction (*See e.g.,* Ferrari et al., "Genetics, " in Hardwood et al, (eds.), Bacillus, Plenum Publishing Corp., pages 57-72, 1989).

As used herein, the terms "transformed" and "stably transformed" refers to a cell that has a non-native (heterologous) polynucleotide sequence integrated into its genome or as an episomal plasmid that is maintained for at least two generations.

As used herein, the term "selectable marker-encoding nucleotide sequence" refers to a nucleotide sequence, which is capable of expression in the host cells and where expression of the selectable marker confers to cells containing the expressed gene the ability to grow in the presence of a corresponding selective agent or lack of an essential nutrient.

As used herein, the terms "selectable marker" and "selective marker" refer to a nucleic acid (*e.g.*, a gene) capable of expression in host cell, which allows for ease of selection of those hosts containing the vector. Examples of such selectable markers include but are not limited to antimicrobials. Thus, the term "selectable marker" refers to genes that provide an indication that a host cell has taken up an incoming DNA of interest or some other reaction has occurred. Typically, selectable markers are genes that confer antimicrobial resistance or a metabolic advantage on the host cell to allow cells containing the exogenous DNA to be distinguished from cells that have not received any exogenous sequence during the transformation. A "residing selectable marker" is one that is located on the chromosome of the microorganism to be transformed. A residing selectable marker encodes a gene that is different from the selectable marker on the transforming DNA construct. Selective markers are well known to those of skill in the art. As indicated above, preferably the marker is an antimicrobial resistant marker (*e.g.*, amp$^R$; phleo$^R$; spec$^R$; kan$^R$; ery$^R$; tet$^R$; cmp$^R$; and neo$^R$ (*See e.g.,* Guerot-Fleury, Gene, 167:335-337, 1995; Palmeros et al., Gene 247:255-264, 2000; and Trieu-Cuot et al., Gene, 23:331-341, 1983). Other markers useful in accordance with the invention include, but are not limited to auxotrophic markers, such as tryptophan; and detection markers, such as β- galactosidase.

As used herein, the term "promoter" refers to a nucleic acid sequence that functions to direct transcription of a downstream gene. In preferred embodiments, the promoter is appropriate to the host cell in which the target gene is being expressed. The promoter, together with other transcriptional and translational regulatory nucleic acid sequences (also termed "control sequences") is necessary to express a given gene. In general, the transcriptional and translational regulatory sequences include, but are not limited to, promoter sequences, ribosomal binding sites, transcriptional start and stop sequences, translational start and stop sequences, and enhancer or activator sequences.

A nucleic acid is "operably linked" when it is placed into a functional relationship with another nucleic acid sequence. For example, DNA encoding a secretory leader (*i.e.,* a signal peptide), is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

As used herein the term "gene" refers to a polynucleotide (*e.g.*, a DNA segment) that encodes a polypeptide and includes regions preceding and following the coding regions as well as intervening sequences (introns) between individual coding segments (exons).

As used herein, "homologous genes" refers to a pair of genes from different, but usually related species, which correspond to each other and which are identical or very similar to each other. The term encompasses genes that are separated by speciation (*i.e.*, the development of new species) (*e.g.*, orthologous genes), as well as genes that have been separated by genetic duplication (*e.g.*, paralogous genes).

As used herein, "ortholog" and "orthologous genes" refer to genes in different species that have evolved from a common ancestral gene *(i.e.,* a homologous gene) by speciation. Typically, orthologs retain the same function during the course of evolution. Identification of orthologs finds use in the reliable prediction of gene function in newly sequenced genomes.

As used herein, "paralog" and "paralogous genes" refer to genes that are related by duplication within a genome. While orthologs retain the same function through the course of evolution, paralogs evolve new functions, even though some functions are often related to the original one. Examples of paralogous genes include, but are not limited to genes encoding trypsin, chymotrypsin, elastase, and thrombin, which are all serine proteinases and occur together within the same species.

As used herein, "homology" refers to sequence similarity or identity, with identity being preferred. This homology is determined using standard techniques known in the art (*See e.g.,* Smith and Waterman, Adv Appl Math, 2:482, 1981; Needleman and Wunsch, J Mol Biol, 48:443, 1970; Pearson and Lipman, Proc Natl Acad Sci USA, 85:2444. 1988; programs such as GAP, BESTFIT, FASTA, and TFASTA in the Wisconsin Genetics Software Package, Genetics Computer Group, Madison, WI; and Devereux et al., Nucl Acid Res, 12:387-395, 1984).

As used herein, an "analogous sequence" is one wherein the function of the gene is essentially the same as the gene based on the *B. amyloliquefaciens* NprE protease. Additionally, analogous genes include at least about 45%, about 50%, about 55%, about 60%, about 65%, about 70%, about 75%, about 80%, about 85%, about 90%, about 95%, about 97%, about 98%, about 99%, or about 100% sequence identity with the sequence of the *B. amyloliquefaciens* NprE protease. In additional embodiments more than one of the above properties applies to the sequence. Analogous sequences are determined by known methods of sequence alignment. A commonly used alignment method is BLAST, although as indicated above and below, there are other methods that also find use in aligning sequences.

One example of a useful algorithm is PILEUP. PILEUP creates a multiple sequence alignment from a group of related sequences using progressive, pair-wise alignments. It can also plot a tree showing the clustering relationships used to create the alignment. PILEUP uses a simplification of the progressive alignment method of Feng and Doolittle (Feng and Doolittle, J Mol Evol, 35:351-360, 1987). The method is similar to that described by Higgins and Sharp (Higgins and Sharp, CABIOS 5:151-153, 1989). Useful PILEUP parameters including a default gap weight of 3.00. a default gap length weight of 0.10, and weighted end gaps.

Another example of a useful algorithm is the BLAST algorithm, described by Altschul *et al.*, (Altschul et al.. J Mol Biol, 215:403-410, 1990; and Karlin et al., Proc Natl Acad Sci USA, 90:5873-5787, 1993). A particularly useful BLAST program is the WU-BLAST-2 program (*See,* Altschul et al., Meth Enzymol, 266:460-480, 1996). WU-BLAST-2 uses several search parameters, most of which are set to the default values. The adjustable parameters are set with the following values: overlap span =1, overlap fraction = 0.125, word threshold (T) = 11. The HSP S and HSP S2 parameters are dynamic values and are established by the program itself depending upon the composition of the particular sequence and composition of the particular database against which the sequence of interest is being searched. However, the values may be adjusted to increase sensitivity. A % amino acid sequence identity value is determined by the number of matching identical residues divided by the total number of residues of the "longer" sequence in the aligned region. The "longer" sequence is the one having the most actual residues in the aligned region (gaps introduced by WU-Blast-2 to maximize the alignment score are ignored).

Thus, "percent (%) nucleic acid sequence identity" is defined as the percentage of nucleotide residues in a candidate sequence that are identical to the nucleotide residues of the starting sequence (*i.e.*, the sequence of interest). A preferred method utilizes the BLASTN module of WU-BLAST-2 set to the default parameters, with overlap span and overlap fraction set to 1 and 0.125, respectively.

As used herein, the term "hybridization" refers to the process by which a strand of nucleic acid joins with a complementary strand through base pairing, as known in the art.

A nucleic acid sequence is considered to be "selectively hybridizable" to a reference nucleic acid sequence if the two sequences specifically hybridize to one another under moderate to high stringency hybridization and wash conditions. Hybridization conditions are based on the melting temperature (Tm) of the nucleic acid binding complex or probe. For example, "maximum stringency" typically occurs at about Tm-5°C (5° below the Tm of the probe); "high stringency" at about 5-10°C below the Tm; "intermediate stringency" at about 10-20°C below the Tm of the probe; and "low stringency" at about 20-25°C below the Tm. Functionally, maximum stringency conditions may be used to identify sequences having strict identity or near-strict identity with the hybridization probe; while intermediate or low stringency hybridization can be used to identify or detect polynucleotide sequence homologs.

Moderate and high stringency hybridization conditions are well known in the art. An example of high stringency conditions includes hybridization at about 42°C in 50% formamide, 5X SSC, 5X Denhardt's solution, 0.5% SDS and 100 μg/ml denatured carrier DNA followed by washing two times in 2X SSC and 0.5% SDS at room temperature and two additional times in 0.1X SSC and 0.5% SDS at 42°C. An example of moderate stringent conditions include an overnight incubation at 37°C in a solution comprising 20% formamide, 5 x SSC (150mM NaCl, 15 mM trisodium citrate), 50 mM sodium phosphate (pH 7.6), 5 x Denhardt's solution, 10% dextran sulfate and 20 mg/ml denatured sheared salmon sperm DNA, followed by washing the filters in 1x SSC at about 37 - 50°C. Those of skill in the art know how to adjust the temperature, ionic strength, etc. as necessary to accommodate factors such as probe length and the like.

As used herein, "recombinant" includes reference to a cell or vector, that has been modified by the introduction of a heterologous nucleic acid sequence or that the cell is derived from a cell so modified. Thus, for example, recombinant cells express genes that are not found in identical form within the native (non-recombinant) form of the cell or express native genes that are otherwise abnormally expressed, under expressed or not expressed at all as a result of deliberate human intervention. "Recombination," "recombining," and generating a "recombined" nucleic acid are generally the assembly of two or more nucleic acid fragments wherein the assembly gives rise to a chimeric gene.

In a preferred embodiment, mutant DNA sequences are generated with site saturation mutagenesis in at least one codon. In another preferred embodiment, site saturation mutagenesis is performed for two or more codons. In a further embodiment, mutant DNA sequences have more than about 50%, more than about 55%, more than about 60%, more than about 65%, more than about 70%, more than about 75%, more than about 80%, more than about 85%, more than about 90%, more than about 95%, or more than about 98% homology with the wild-type sequence. In alternative embodiments, mutant DNA is generated *in vivo* using any known mutagenic procedure such as, for example, radiation, nitrosoguanidine and the like. The desired DNA sequence is then isolated and used in the methods provided herein.

As used herein, the term "target sequence" refers to a DNA sequence in the host cell that encodes the sequence where it is desired for the incoming sequence to be inserted into the host cell genome. In some embodiments, the target sequence encodes a functional wild-type gene or operon, while in other embodiments the target sequence encodes a functional mutant gene or operon, or a non-functional gene or operon.

As used herein, a "flanking sequence" refers to any sequence that is either upstream or downstream of the sequence being discussed (*e.g.*, for genes A-B-C, gene B is flanked by the A and C gene sequences). In a preferred embodiment, the incoming sequence is flanked by a homology box on each side. In another embodiment, the incoming sequence and the homology boxes comprise a unit that is flanked by stuffer sequence on each side. In some embodiments, a flanking sequence is present on only a single side (either 3' or 5'), but in preferred embodiments, it is on each side of the sequence being flanked. In some embodiments, a flanking sequence is present on only a single side (either 3' or 5'), while in preferred embodiments, it is present on each side of the sequence being flanked.

As used herein, the term "stuffer sequence" refers to any extra DNA that flanks homology boxes (typically vector sequences). However, the term encompasses any non-homologous DNA sequence. Not to be limited by any theory, a stuffer sequence provides a noncritical target for a cell to initiate DNA uptake.

As used herein, the terms "amplification" and "gene amplification" refer to a process by which specific DNA sequences are disproportionately replicated such that the amplified gene becomes present in a higher copy number than was initially present in the genome. In some embodiments, selection of cells by growth in the presence of a drug (*e.g.*, an inhibitor of an inhibitable enzyme) results in the amplification of either the endogenous gene encoding the gene product required for growth in the presence of the drug or by amplification of exogenous (i.e., input) sequences encoding this gene product, or both.

"Amplification" is a special case of nucleic acid replication involving template specificity. It is to be contrasted with non-specific template replication (*i.e.*, replication that is template-dependent but not dependent on a specific template). Template specificity is here distinguished from fidelity of replication (*i.e.*, synthesis of the proper polynucleotide sequence) and nucleotide (ribo- or deoxyribo-) specificity. Template specificity is frequently described in terms of "target" specificity. Target sequences are "targets" in the sense that they are sought to be sorted out from other nucleic acid. Amplification techniques have been designed primarily for this sorting out.

As used herein, the term "co-amplification" refers to the introduction into a single cell of an amplifiable marker in conjunction with other gene sequences (*i.e.*, comprising one or more non-selectable genes such as those contained within an expression vector) and the application of appropriate selective pressure such that the cell amplifies both the amplifiable marker and the other, non-selectable gene sequences. The amplifiable marker may be physically linked to the other gene sequences or alternatively two separate pieces of DNA, one containing the amplifiable marker and the other containing the non-selectable marker, may be introduced into the same cell.

As used herein, the terms "amplifiable marker," "amplifiable gene," and "amplification vector" refer to a gene or a vector encoding a gene, which permits the amplification of that gene under appropriate growth conditions.

"Template specificity" is achieved in most amplification techniques by the choice of enzyme. Amplification enzymes are enzymes that, under conditions they are used, will process only specific sequences of nucleic acid in a heterogeneous mixture of nucleic acid. For example, in the case of Qβ replicase, MDV-1 RNA is the specific template for the replicase (*See e.g.,* Kacian et al., Proc Natl Acad Sci USA 69:3038, 1972) and other nucleic acids are not replicated by this amplification enzyme. Similarly, in the case of T7 RNA polymerase, this amplification enzyme has a stringent specificity for its own promoters (*See,* Chamberlin et al., Nature 228:227, 1970). In the case of T4 DNA ligase, the enzyme will not ligate the two oligonucleotides or polynucleotides, where there is a mismatch between the oligonucleotide or polynucleotide substrate and the template at the ligation junction (*See,* Wu and Wallace, Genomics 4:560, 1989). Finally, *Taq* and *Pfu* polymerases, by virtue of their ability to function at high temperature, are found to display high specificity for the sequences bounded and thus defined by the primers; the high temperature results in thermodynamic conditions that favor primer hybridization with the target sequences and not hybridization with non-target sequences.

As used herein, the term "amplifiable nucleic acid" refers to nucleic acids, which may be amplified by any amplification method. It is contemplated that "amplifiable nucleic acid" will usually comprise "sample template."

As used herein, the term "sample template" refers to nucleic acid originating from a sample, which is analyzed for the presence of "target" (defined below). In contrast, "background template" is used in reference to nucleic acid other than sample template, which may or may not be present in a sample. Background template is most often inadvertent. It may be the result of carryover, or it may be due to the presence of nucleic acid contaminants sought to be purified away from the sample. For example, nucleic acids from organisms other than those to be detected may be present as background in a test sample.

As used herein, the term "primer" refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, (*i.e.*, in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer and the use of the method.

As used herein, the term "probe" refers to an oligonucleotide (i.e., a sequence of nucleotides), whether occurring naturally as in a purified restriction digest or produced synthetically, recombinantly or by PCR amplification, which is capable of hybridizing to another oligonucleotide of interest. A probe may be single-stranded or double-stranded. Probes are useful in the detection, identification and isolation of particular gene sequences. It is contemplated that any probe used in the present invention will be labeled with any "reporter molecule." so that is detectable in any detection system, including, but not limited to enzyme (*e.g.*, ELISA, as well as enzyme-based histochemical assays), fluorescent, radioactive, and luminescent systems. It is not intended that the present invention be limited to any particular detection system or label.

As used herein, the term "target," when used in reference to the polymerase chain reaction, refers to the region of nucleic acid bounded by the primers used for polymerase chain reaction. Thus, the "target" is sought to be sorted out from other nucleic acid sequences. A "segment" is defined as a region of nucleic acid within the target sequence.

As used herein, the term "polymerase chain reaction" ("PCR") refers to the methods of U.S. Patent Nos. 4.683.195 4.683.202. and 4.965.188, which include methods for increasing the concentration of a segment of a target sequence in a mixture of genomic DNA without cloning or purification. This process for amplifying the target sequence consists of introducing a large excess of two oligonucleotide primers to the DNA mixture containing the desired target sequence, followed by a precise sequence of thermal cycling in the presence of a DNA polymerase. The two primers are complementary to their respective strands of the double stranded target sequence. To effect amplification, the mixture is denatured and the primers then annealed to their complementary sequences within the target molecule. Following annealing, the primers are extended with a polymerase so as to form a new pair of complementary strands. The steps of denaturation, primer annealing and polymerase extension can be repeated many times (*i.e.*, denaturation, annealing and extension constitute one "cycle"; there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplified".

As used herein, the term "amplification reagents" refers to those reagents (deoxyribonucleotide triphosphates, buffer, etc.), needed for amplification except for primers, nucleic acid template and the amplification enzyme. Typically, amplification reagents along with other reaction components are placed and contained in a reaction vessel (test tube, microwell, etc.).

With PCR. it is possible to amplify a single copy of a specific target sequence in genomic DNA to a level detectable by several different methodologies (*e.g.*, hybridization with a labeled probe; incorporation of biotinylated primers followed by avidin-enzyme conjugate detection; incorporation of $^{32}$P-labeled deoxynucleotide triphosphates, such as dCTP or dATP, into the amplified segment). In addition to genomic DNA, any oligonucleotide or polynucleotide sequence can be amplified with the appropriate set of primer molecules. In particular, the amplified segments created by the PCR process itself are, themselves, efficient templates for subsequent PCR amplifications.

As used herein, the terms "PCR product," "PCR fragment," and "amplification product" refer to the resultant mixture of compounds after two or more cycles of the PCR steps of denaturation, annealing and extension are complete. These terms encompass the case where there has been amplification of one or more segments of one or more target sequences.

As used herein, the term "RT-PCR" refers to the replication and amplification of RNA sequences. In this method, reverse transcription is coupled to PCR, most often using a one enzyme procedure in which a thermostable polymerase is employed, as described in U.S. Patent No. 5,322,770. In RT-PCR. the RNA template is converted to cDNA due to the reverse transcriptase activity of the polymerase, and then amplified using the polymerizing activity of the polymerase (*i.e.*, as in other PCR methods).

As used herein, the terms "restriction endonucleases" and "restriction enzymes" refer to bacterial enzymes, each of which cut double-stranded DNA at or near a specific nucleotide sequence.

A "restriction site" refers to a nucleotide sequence recognized and cleaved by a given restriction endonuclease and is frequently the site for insertion of DNA fragments. In certain embodiments of the invention restriction sites are engineered into the selective marker and into 5' and 3' ends of the DNA construct.

As used herein, the term "chromosomal integration" refers to the process whereby an incoming sequence is introduced into the chromosome of a host cell. The homologous regions of the transforming DNA align with homologous regions of the chromosome. Subsequently, the sequence between the homology boxes is replaced by the incoming sequence in a double crossover (*i.e.*, homologous recombination). In some embodiments of the present invention, homologous sections of an inactivating chromosomal segment of a DNA construct align with the flanking homologous regions of the indigenous chromosomal region of the *Bacillus* chromosome. Subsequently, the indigenous chromosomal region is deleted by the DNA construct in a double crossover (*i.e.*, homologous recombination).

"Homologous recombination" means the exchange of DNA fragments between two DNA molecules or paired chromosomes at the site of identical or nearly identical nucleotide sequences. In a preferred embodiment, chromosomal integration is homologous recombination.

"Homologous sequences" as used herein means a nucleic acid or polypeptide sequence having about 100%, about 99%, about 98%, about 97%, about 96%, about 95%, about 94%, about 93%, about 92%, about 91%, about 90%, about 88%, about 85%, about 80%, about 75%, or about 70% sequence identity to another nucleic acid or polypeptide sequence when optimally aligned for comparison. In some embodiments, homologous sequences have between about 85% and about 100% sequence identity, while in other embodiments there is between about 90% and about 100% sequence identity, and in more preferred embodiments, there is about 95% and about 100% sequence identity.

As used herein "amino acid" refers to peptide or protein sequences or portions thereof. The terms "protein," "peptide," and "polypeptide" are used interchangeably.

As used herein, the term "heterologous protein" refers to a protein or polypeptide that does not naturally occur in the host cell. Examples of heterologous proteins include enzymes such as hydrolases including proteases. In some embodiments, the gene encoding the proteins are naturally occurring genes, while in other embodiments, mutated and/or synthetic genes are used.

As used herein, "homologous protein" refers to a protein or polypeptide native or naturally occurring in a cell. In preferred embodiments, the cell is a Gram-positive cell, while in particularly preferred embodiments the cell is a *Bacillus* host cell. In alternative embodiments the homologous protein is a native protein produced by other organisms, including but not limited to *E. coli, Streptomyces, Trichoderma,* and *Aspergillus.* The invention encompasses host cells producing the homologous protein via recombinant DNA technology.

As used herein, an "operon region" comprises a group of contiguous genes that are transcribed as a single transcription unit from a common promoter, and are thereby subject to co-regulation. In some embodiments, the operon includes a regulator gene. In most preferred embodiments, operons that are highly expressed as measured by RNA levels, but have an unknown or unnecessary function are used.

As used herein, an "antimicrobial region" is a region containing at least one gene that encodes an antimicrobial protein.

A polynucleotide is said to "encode" an RNA or a polypeptide if, in its native state or when manipulated by methods known to those of skill in the art, it can be transcribed and/or translated to produce the RNA, the polypeptide or a fragment thereof. The anti-sense strand of such a nucleic acid is also said to encode the sequences.

As is known in the art, a DNA can be transcribed by an RNA polymerase to produce RNA, but an RNA can be reverse transcribed by reverse transcriptase to produce a DNA. Thus a DNA can encode a RNA and vice versa.

The term "regulator segment" or "regulatory sequence" or "expression control sequence" refers to a polynucleotide sequence of DNA that is operatively linked with a polynucleotide sequence of DNA that encodes the amino acid sequence of a polypeptide chain to effect the expression of the encoded amino acid sequence. The regulatory sequence can inhibit, repress, or promote the expression of the operably linked polynucleotide sequence encoding the amino acid.

"Host strain" or "host cell" refers to a suitable host for an expression vector comprising DNA according to the present invention.

An enzyme is "overexpressed" in a host cell if the enzyme is expressed in the cell at a higher level that the level at which it is expressed in a corresponding wild-type cell.

The terms "protein" and "polypeptide" are used interchangeability herein. The 3-letter code for amino acids as defined in conformity with the IUPAC-IUB Joint Commission on Biochemical Nomenclature (JCBN) is used through out this disclosure. It is also understood that a polypeptide may be coded for by more than one nucleotide sequence due to the degeneracy of the genetic code:

A "prosequence" is an amino acid sequence between the signal sequence and mature protease that is necessary for the secretion of the protease. Cleavage of the pro sequence will result in a mature active protease.

The term "signal sequence" or "signal peptide" refers to any sequence of nucleotides and/or amino acids that participate in the secretion of the mature or precursor forms of the protein. This definition of signal sequence is a functional one, meant to include all those amino acid sequences encoded by the N-terminal portion of the protein gene, which participate in the effectuation of the secretion of protein. They are often, but not universally, bound to the N-terminal portion of a protein or to the N-terminal portion of a precursor protein. The signal sequence may be endogenous or exogenous. The signal sequence may be that normally associated with the protein (e.g., protease), or may be from a gene encoding another secreted protein. One exemplary exogenous signal sequence comprises the first seven amino acid residues of the signal sequence from *Bacillus subtilis* subtilisin fused to the remainder of the signal sequence of the subtilisin from *Bacillus lentus* (ATCC 21536).

The term "hybrid signal sequence" refers to signal sequences in which part of sequence is obtained from the expression host fused to the signal sequence of the gene to be expressed. In some embodiments, synthetic sequences are utilized.

The term "mature" form of a protein or peptide refers to the final functional form of the protein or peptide. To exemply, a mature form of the NprE protease of the present invention at least includes the amino acid sequence of SEQ ID NO:3.

The term "precursor" form of a protein or peptide refers to a mature form of the protein having a prosequence operably linked to the amino or carbonyl terminus of the protein. The precursor may also have a "signal" sequence operably linked, to the amino terminus of the prosequence. The precursor may also have additional polynucleotides that are involved in post-translational activity (*e.g.*, polynucleotides cleaved therefrom to leave the mature form of a protein or peptide).

"Naturally occurring enzyme" refers to an enzyme having the unmodified amino acid sequence identical to that found in nature. Naturally occurring enzymes include native enzymes those enzymes naturally expressed or found in the particular microorganism.

The terms "derived from" and "obtained from" refer to not only a protease produced or producible by a strain of the organism in question, but also a protease encoded by a DNA sequence isolated from such strain and produced in a host organism containing such DNA sequence. Additionally, the term refers to a protease that is encoded by a DNA sequence of synthetic and/or cDNA origin and which has the identifying characteristics of the protease in question. To exemplify, "proteases derived from *Bacillus sp.*" refers to those enzymes having proteolytic activity which are naturally-produced by *Bacillus sp.,* as well as to neutral metalloproteases like those produced by *Bacillus sp.* sources but which through the use of genetic engineering techniques are produced by non-*Bacillus amyloliquefaciens* organisms transformed with a nucleic acid encoding said neutral metalloproteases.

A "derivative" within the scope of this definition generally retains the characteristic proteolytic activity observed in the wild-type, native or parent form to the extent that the derivative is useful for similar purposes as the wild-type, native or parent form. Functional derivatives of neutral metalloprotease encompass naturally occurring, synthetically or recombinantly produced peptides or peptide fragments having the general characteristics of the neutral metalloprotease of the present invention.

The term "functional derivative" refers to a derivative of a nucleic acid having the functional characteristics of a nucleic acid encoding a neutral metalloprotease. Functional derivatives of a nucleic acid, which encode neutral metalloprotease of the present invention encompass naturally occurring, synthetically or recombinantly produced nucleic acids or fragments and encode neutral metalloprotease characteristic of the present invention. Wild type nucleic acid encoding neutral metalloprotease according to the invention include naturally occurring alleles and homologues based on the degeneracy of the genetic code known in the art.

The term "identical" in the context of two nucleic acids or polypeptide sequences refers to the residues in the two sequences that are the same when aligned for maximum correspondence, as measured using one of the following sequence comparison or analysis algorithms.

The term "optimal alignment" refers to the alignment giving the highest percent identity score.

"Percent sequence identity," "percent amino acid sequence identity," "percent gene sequence identity," and/or "percent nucleic acid/polynucleotide sequence identity," with respect to two amino acid, polynucleotide and/or gene sequences (as appropriate), refer to the percentage of residues that are identical in the two sequences when the sequences are optimally aligned. Thus, "80% amino acid sequence identity" means that 80% of the amino acids in two optimally aligned polypeptide sequences are identical.

The phrase "substantially identical" in the context of two nucleic acids or polypeptides thus refers to a polynucleotide or polypeptide that comprising at least about 70% sequence identity, preferably at least about 75%, preferably at least about 80%, preferably at least about 85%, preferably at least about 90%, preferably at least about 95%, preferably at least about 97% , preferably at least about 98%, and preferably at least about 99% sequence identity as compared to a reference sequence using the programs or algorithms (*e.g.,* BLAST, ALIGN, CLUSTAL) using standard parameters. One indication that two polypeptides are substantially identical is that the first polypeptide is immunologically cross-reactive with the second polypeptide. Typically, polypeptides that differ by conservative amino acid substitutions are immunologically cross-reactive. Thus, a polypeptide is substantially identical to a second polypeptide, for example, where the two peptides differ only by a conservative substitution. Another indication that two nucleic acid sequences are substantially identical is that the two molecules hybridize to each other under stringent conditions (*e.g.*, within a range of medium to high stringency).

The term "isolated" or "purified" refers to a material that is removed from its original environment (e.g., the natural environment if it is naturally occurring). For example, the material is said to be "purified" when it is present in a particular composition in a higher or lower concentration than exists in a naturally occurring or wild type organism or in combination with components not normally present upon expression from a naturally occurring or wild type organism. For example, a naturally-occurring polynucleotide or polypeptide present in a living animal is not isolated, but the same polynucleotide or polypeptide, separated from some or all of the coexisting materials in the natural system, is isolated. Such polynucleotides could be part of a vector, and/or such polynucleotides or polypeptides could be part of a composition, and still be isolated in that such vector or composition is not part of its natural environment. In preferred embodiments, a nucleic acid or protein is said to be purified, for example, if it gives rise to essentially one band in an electrophoretic gel or blot.

The term "isolated", when used in reference to a DNA sequence, refers to a DNA sequence that has been removed from its natural genetic milieu and is thus free of other extraneous or unwanted coding sequences, and is in a form suitable for use within genetically engineered protein production systems. Such isolated molecules are those that are separated from their natural environment and include cDNA and genomic clones. Isolated DNA molecules of the present invention are free of other genes with which they are ordinarily associated, but may include naturally occurring 5' and 3' untranslated regions such as promoters and terminators. The identification of associated regions will be evident to one of ordinary skill in the art *(See e.g.,* Dynan and Tijan, Nature 316:774-78, 1985). The term "an isolated DNA sequence" is alternatively referred to as "a cloned DNA sequence".

The term "isolated," when used in reference to a protein, refers to a protein that is found in a condition other than its native environment. In a preferred form, the isolated protein is substantially free of other proteins, particularly other homologous proteins. An isolated protein is more than about 10% pure, preferably more than about 20% pure, and even more preferably more than about 30% pure, as determined by SDS-PAGE. Further aspects of the invention encompass the protein in a highly purified form (*i.e.,* more than about 40% pure, more than about 60% pure. more than about 80% pure, more than about 90% pure, more than about 95% pure, more than about 97% pure, and even more than about 99% pure), as determined by SDS-PAGE.

The following cassette mutagenesis method may be used to facilitate the construction of the enzyme variants of the present invention, although other methods may be used. First, as described herein, a naturally-occurring gene encoding the enzyme is obtained and sequenced in whole or in part. Then, the sequence is scanned for a point at which it is desired to make a mutation (deletion, insertion or substitution) of one or more amino acids in the encoded enzyme. The sequences flanking this point are evaluated for the presence of restriction sites for replacing a short segment of the gene with an oligonucleotide pool which when expressed will encode various mutants. Such restriction sites are preferably unique sites within the protein gene so as to facilitate the replacement of the gene segment. However, any convenient restriction site that is not overly redundant in the enzyme gene may be used, provided the gene fragments generated by restriction digestion can be reassembled in proper sequence. If restriction sites are not present at locations within a convenient distance from the selected point (from 10 to 15 nucleotides), such sites are generated by substituting nucleotides in the gene in such a fashion that neither the reading frame nor the amino acids encoded are changed in the final construction. Mutation of the gene in order to change its sequence to conform to the desired sequence is accomplished by M13 primer extension in accord with generally known methods. The task of locating suitable flanking regions and evaluating the needed changes to arrive at two convenient restriction site sequences is made routine by the redundancy of the genetic code, a restriction enzyme map of the gene and the large number of different restriction enzymes. Note that if a convenient flanking restriction site is available, the above method need be used only in connection with the flanking region that does not contain a site.

Once the naturally-occurring DNA and/or synthetic DNA is cloned, the restriction sites flanking the positions to be mutated are digested with the cognate restriction enzymes and a plurality of end termini-complementary oligonucleotide cassettes are ligated into the gene. The mutagenesis is simplified by this method because all of the oligonucleotides can be synthesized so as to have the same restriction sites, and no synthetic linkers are necessary to create the restriction sites.

As used herein, "corresponding to," refers to a residue at the enumerated position in a a protein or peptide, or a residue that is analogous, homologous, or equivalent to an enumerated residue in a protein or peptide.

As used herein, "corresponding region," generally refers to an analogous position along related proteins or a parent protein.

As used herein, the term, "combinatorial mutagenesis" refers to methods in which libraries of variants of a starting sequence are generated. In these libraries, the variants contain one or several mutations chosen from a predefined set of mutations. In addition, the methods provide means to introduce random mutations, which were not members of the predefined set of mutations. In some embodiments, the methods include those set forth in U.S. Application No. 09/699.250. filed October 26, 2000. In alternative embodiments, combinatorial mutagenesis methods encompass commercially available kits (*e.g.*, QUIKCHANGE® Multisite, Stratagene, San Diego. CA).

As used herein, the term "library of mutants" refers to a population of cells which are identical in most of their genome but include different homologues of one or more genes. Such libraries can be used, for example, to identify genes or operons with improved traits.

As used herein, the terms "starting gene" and "parent gene" refer to a gene of interest that encodes a protein of interest that is to be improved and/or changed using the present invention.

As used herein, the terms "multiple sequence alignment" and "MSA" refer to the sequences of multiple homologs of a starting gene that are aligned using an algorithm (e.g., Clustal W).

As used herein, the terms "consensus sequence" and "canonical sequence" refer to an archetypical amino acid sequence against which all variants of a particular protein or sequence of interest are compared. The terms also refer to a sequence that sets forth the nucleotides that are most often present in a DNA sequence of interest. For each position of a gene, the consensus sequence gives the amino acid that is most abundant in that position in the MSA.

As used herein, the term "consensus mutation" refers to a difference in the sequence of a starting gene and a consensus sequence. Consensus mutations are identified by comparing the sequences of the starting gene and the consensus sequence obtained from a MSA. In some embodiments, consensus mutations are introduced into the starting gene such that it becomes more similar to the consensus sequence. Consensus mutations also include amino acid changes that change an amino acid in a starting gene to an amino acid that is more frequently found in an MSA at that position relative to the frequency of that amino acid in the starting gene. Thus, the term consensus mutation comprises all single amino acid changes that replace an amino acid of the starting gene with an amino acid that is more abundant than the amino acid in the MSA.

The terms "modified sequence" and "modified genes" are used interchangeably herein to refer to a sequence that includes a deletion, insertion or interruption of naturally occurring nucleic acid sequence. In some preferred embodiments, the expression product of the modified sequence is a truncated protein (*e.g.,* if the modification is a deletion or interruption of the sequence). In some particularly preferred embodiments, the truncated protein retains biological activity. In alternative embodiments, the expression product of the modified sequence is an elongated protein (e.g., modifications comprising an insertion into the nucleic acid sequence). In some embodiments, an insertion leads to a truncated protein (*e.g.,* when the insertion results in the formation of a stop codon). Thus, an insertion may result in either a truncated protein or an elongated protein as an expression product.

As used herein, the terms "mutant sequence" and "mutant gene" are used interchangeably and refer to a sequence that has an alteration in at least one codon occurring in a host cell's wild-type sequence. The expression product of the mutant sequence is a protein with an altered amino acid sequence relative to the wild-type. The expression product may have an altered functional capacity (*e.g*., enhanced enzymatic activity).

The terms "mutagenic primer" or "mutagenic oligonucleotide" (used interchangeably herein) are intended to refer to oligonucleotide compositions which correspond to a portion of the template sequence and which are capable of hybridizing thereto. With respect to mutagenic primers, the primer will not precisely match the template nucleic acid, the mismatch or mismatches in the primer being used to introduce the desired mutation into the nucleic acid library. As used herein, "non-mutagenic primer" or "non-mutagenic oligonucleotide" refers to oligonucleotide compositions that match precisely to the template nucleic acid. In one embodiment of the invention, only mutagenic primers are used. In another preferred embodiment of the invention, the primers are designed so that for at least one region at which a mutagenic primer has been included, there is also non-mutagenic primer included in the oligonucleotide mixture. By adding a mixture of mutagenic primers and non-mutagenic primers corresponding to at least one of the mutagenic primers, it is possible to produce a resulting nucleic acid library in which a variety of combinatorial mutational patterns are presented. For example, if it is desired that some of the members of the mutant nucleic acid library retain their parent sequence at certain positions while other members are mutant at such sites, the non-mutagenic primers provide the ability to obtain a specific level of non-mutant members within the nucleic acid library for a given residue. The methods of the invention employ mutagenic and non-mutagenic oligonucleotides which are generally between 10-50 bases in length, more preferably about 15-45 bases in length. However, it may be necessary to use primers that are either shorter than 10 bases or longer than 50 bases to obtain the mutagenesis result desired. With respect to corresponding mutagenic and non-mutagenic primers, it is not necessary that the corresponding oligonucleotides be of identical length, but only that there is overlap in the region corresponding to the mutation to be added.

Primers may be added in a pre-defined ratio according to the present invention. For example, if it is desired that the resulting library have a significant level of a certain specific mutation and a lesser amount of a different mutation at the same or different site, by adjusting the amount of primer added, it is possible to produce the desired biased library. Alternatively, by adding lesser or greater amounts of non-mutagenic primers, it is possible to adjust the frequency with which the corresponding mutation(s) are produced in the mutant nucleic acid library.

The terms "wild-type sequence," or "wild-type gene" are used interchangeably herein, to refer to a sequence that is native or naturally occurring in a host cell. In some embodiments, the wild-type sequence refers to a sequence of interest that is the starting point of a protein-engineering project. The wild-type sequence may encode either a homologous or heterologous protein. A homologous protein is one the host cell would produce without intervention. A heterologous protein is one that the host cell would not produce but for the intervention.

The term "oxidation stable" refers to proteases of the present invention that retain a specified amount of enzymatic activity over a given period of time under conditions prevailing during the proteolytic, hydrolyzing, cleaning or other process of the invention, for example while exposed to or contacted with bleaching agents or oxidizing agents. In some embodiments, the proteases retain at least about 50%, about 60%, about 70%, about 75%, about 80%, about 85%, about 90%, about 92%, about 95%, about 96%, about 97%, about 98%, or about 99% proteolytic activity after contact with a bleaching or oxidizing agent over a given time period, for example, at least 1 minute, 3 minutes, 5 minutes, 8 minutes. 12 minutes, 16 minutes, 20 minutes, etc.

The term "chelator stable" refers to proteases of the present invention that retain a specified amount of enzymatic activity over a given period of time under conditions prevailing during the proteolytic, hydrolyzing, cleaning or other process of the invention, for example while exposed to or contacted with chelating agents. In some embodiments, the proteases retain at least about 50%, about 60%, about 70%, about 75%, about 80%, about 85%, about 90%, about 92%, about 95%, about 96%, about 97%, about 98% or about 99% proteolytic activity after contact with a chelating agent over a given time period, for example, at least 10 minutes, 20 minutes, 40 minutes, 60 minutes, 100 minutes, etc.

The terms "thermally stable" and "thermostable" refer to proteases of the present invention that retain a specified amount of enzymatic activity after exposure to identified temperatures over a given period of time under conditions prevailing during the proteolytic, hydrolyzing, cleaning or other process of the invention, for example while exposed altered temperatures. Altered temperatures include increased or decreased temperatures. In some embodiments, the proteases retain at least about 50%, about 60%, about 70%, about 75%, about 80%, about 85%, about 90%, about 92%, about 95%, about 96%, about 97%, about 98%, or about 99% proteolytic activity after exposure to altered temperatures over a given time period, for example, at least 60 minutes, 120 minutes, 180 minutes, 240 minutes, 300 minutes, etc.

As used herein, the term "chemical stability" refers to the stability of a protein (*e.g.,* an enzyme) towards chemicals that adversely affect its activity. In some embodiments, such chemicals include, but are not limited to hydrogen peroxide, peracids, anionic detergents, cationic detergents, non-ionic detergents, chelants, etc. However, it is not intended that the present invention be limited to any particular chemical stability level nor range of chemical stability. In particular, the terms "detergent stable" and "LAS stable" refer to proteases of the present invention that retain a specified amount of enzymatic activity after exposure to a detergent composition over a given period of time under conditions prevailing during the proteolytic, hydrolyzing, cleaning or other process of the invention. In some embodiments, the proteases retain at least about 50%, about 60%, about 70%, about 75%, about 80%, about 85%, about 90%, about 92%, about 95%, about 96%, about 97%, about 98%, or about 99% proteolytic activity after exposure to detergent over a given time period, for example, at least 60 minutes, 120 minutes, 180 minutes, 240 minutes, 300 minutes, etc.

The term "enhanced stability" in the context of an oxidation, chelator, thermal and/or pH stable protease refers to a higher retained proteolytic activity over time as compared to other neutral metalloproteases and/or wild-type enzymes.

The term "diminished stability" in the context of an oxidation, chelator, thermal and/or pH stable protease refers to a lower retained proteolytic activity over time as compared to other neutral metalloproteases and/or wild-type enzymes.

As used herein, the term "cleaning composition" includes, unless otherwise indicated, granular or powder-form all-purpose or "heavy-duty" washing agents, especially cleaning detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use: liquid cleaning and disinfecting agents, including antibacterial hand-wash types, cleaning bars, mouthwashes, denture cleaners, car or carpet shampoos, bathroom cleaners: hair shampoos and hair-rinses; shower gels and foam baths and metal cleaners; as well as cleaning auxiliaries such as bleach additives and "stain-stick" or pre-treat types.

Unless otherwise noted, all component or composition levels are in reference to the active level of that component or composition, and are exclusive of impurities, for example, residual solvents or by-products, which may be present in commercially available sources.

Enzyme components weights are based on total active protein. All percentages and ratios are calculated by weight unless otherwise indicated. All percentages and ratios are calculated based on the total composition unless otherwise indicated.

The term "cleaning activity" refers to the cleaning performance achieved by the protease under conditions prevailing during the proteolytic, hydrolyzing, cleaning or other process of the invention. In some embodiments, cleaning performance is determined by the application of various cleaning assays concerning enzyme sensitive stains, for example grass, blood, milk, or egg protein as determined by various chromatographic, spectrophotometric or other quantitative methodologies after subjection of the stains to standard wash conditions. Exemplary assays include, but are not limited to those described in WO 99/34011, and U.S. Patent No. 6.605.458, as well as those methods included in the Examples.

The term "cleaning effective amount" of a protease refers to the quantity of protease described hereinbefore that achieves a desired level of enzymatic activity in a specific cleaning composition. Such effective amounts are readily ascertained by one of ordinary skill in the art and are based on many factors, such as the particular protease used, the cleaning application, the specific composition of the cleaning composition, and whether a liquid or dry (*e.g.,* granular, bar) composition is required, etc.

The term "cleaning adjunct materials" as used herein, means any liquid, solid or gaseous material selected for the particular type of cleaning composition desired and the form of the product (*e.g.*, liquid, granule, powder, bar, paste, spray, tablet, gel; or foam composition), which materials are also preferably compatible with the protease enzyme used in the composition. In some embodiments, granular compositions are in "compact" form, while in other embodiments, the liquid compositions are in a "concentrated" form.

As used herein, a "low detergent concentration" system includes detergents where less than about 800 ppm of detergent components are present in the wash water. Japanese detergents are typically considered low detergent concentration systems, as they have usually have approximately 667 ppm of detergent components present in the wash water.

As used herein, a "medium detergent concentration" systems includes detergents wherein between about 800 ppm and about 2000ppm of detergent components are present in the wash water. North American detergents are generally considered to be medium detergent concentration systems as they have usually approximately 975 ppm of detergent components present in the wash water. Brazilian detergents typically have approximately 1500 ppm of detergent components present in the wash water.

As used herein, "high detergent concentration" systems includes detergents wherein greater than about 2000 ppm of detergent components are present in the wash water. European detergents are generally considered to be high detergent concentration systems as they have approximately 3000-8000 ppm of detergent components in the wash water.

As used herein, "fabric cleaning compositions" include hand and machine laundry detergent compositions including laundry additive compositions and compositions suitable for use in the soaking and/or pretreatment of stained fabrics (e.g., clothes, linens, and other textile materials).

As used herein, "non-fabric cleaning compositions" include non-textile (i.e., fabric) surface cleaning compositions, including but not limited to dishwashing detergent compositions, oral cleaning compositions, denture cleaning compositions, and personal cleansing compositions.

The "compact" form of the cleaning compositions herein is best reflected by density and, in terms of composition, by the amount of inorganic filler salt. Inorganic filler salts are conventional ingredients of detergent compositions in powder form. In conventional detergent compositions, the filler salts are present in substantial amounts, typically 17-35% by weight of the total composition. In contrast, in compact compositions, the filler salt is present in amounts not exceeding 15% of the total composition. In some embodiments, the filler salt is present in amounts that do not exceed 10%, or more preferably, 5%, by weight of the composition. In some embodiments, the inorganic filler salts are selected from the alkali and alkaline-earth-metal salts of sulfates and chlorides. A preferred filler salt is sodium sulfate.

## DETAILED DESCRIPTION OF THE PRESENT INVENTION

Neutral metalloendopeptidases (i.e., neutral metalloproteases) (EC 3.4.24.4) belong to a protease class that has an absolute requirement for zinc ions for catalytic activity. These enzymes are optimally active at neutral pH and are in the 30 to 40 kDa size range. Neutral metalloproteases bind between two and four calcium ions that contribute to the structural stability of the protein. The bound metal ion at the active site of metalloproteases is an essential feature that allows the activation of a water molecule. The water molecule then functions as the nucleophile and cleaves the carbonyl group of the peptide bond.

The neutral zinc-binding metalloprotease family includes the bacterial enzyme thermolysin, and thermolysin-like proteases ("TLPs"), as well as carboxypeptidase A (a digestive enzyme), and the matrix metalloproteases that catalyze the reactions in tissue remodeling and degradation. The only well characterized of these proteases, with respect to stability and function, is thermolysin and its variants (TLPs). Indeed, much research has been focused on the engineering Bacillus subtilis neutral proteases to increase the thermal stability of the enzyme (See e.g., Vriend et al., In, Tweel et al. (eds), Stability and Stabilization of enzymes, Elsevier, pp. 93-99 [1993]).

Most effort has been focused on increasing the stability of the protease by altering structural determinants identified through the use of molecular modeling suggested to prevent local unfolding processes that would result in autolysis of the protein and cause the neutral protease to denature at high temperatures (See e.g., van den Burg et al., in Hopsu-Havu et al., (eds), Proteolysis in Cell Functions Manipulating the Autolytic Pathway of a Bacillus Protease. Biomedical and Health Research Vol. 13, IOS Press [1997] p. 576).

Compositions and methods to engineer neutral metalloproteases with improved characteristics are provided herein. As indicated herein, calcium ions have been reported for other proteases such as thermolysin to prevent autolysis. The B. stearothermophilus neutral protease has been stabilized against autolysis and proteolytic degradation by addition of calcium (See, Dürrschmidt et al., FEBS J., 272:1523-1534 [2005]).

Indeed, the present invention provides compositions and methods suitable for the engineering of neutral metalloproteases that are independent of calcium in order to maintain their structural stability. In some embodiments, engineering prevents the local unfolding in a particular secondary structural element that may prevent proteolysis.

Natural and engineered proteases, such as subtilisin are often expressed in Bacillus subtilis and several have been applied in detergent formulations to remove proteinaceous stains. Others have been applied for example in the baking industry (e.g., thermolysin from Bacillus thermoproteolyticus; See e.g., Galante and Formantici, Curr. Organic Chem.. 7, 1399-1422 [2003]). In general, the serine proteases have been more widely utilized in detergents, at least partially due to the relative ease with which these proteases can be stabilized.

Indeed, metalloproteases are less frequently used in industry, and particularly in the detergent industry for a number of reasons. These enzymes involve more complex protein systems, as the enzymes have the absolute requirement for calcium and zinc ions for stability and function, respectively. Further, the detergent solution as well as the water used in the laundry process often contains components that often interfere with the binding of the ions by the enzyme or chelate these ions, resulting in a decrease or loss of proteolytic function and destabilization of the protease.

As determined during development of the present invention, the autolysis model for NprE in the presence of citrate may be represented by the equation:

$$N \leftrightharpoons I \rightarrow A_P,$$

where N is the native form. I represents a partially-folded intermediate and Ap is the autolysed protease. For NprE, citrate most likely destabilizes the protein structure by removing calcium ions resulting in a partially unfolded intermediate (I) state, making loops and surface residues susceptible to autolysis. The generation of the autolysed protein fragments would be rapid and of high order. Stabilizing substitutions at or near these regions resulted in NprE variants that are less susceptible to autolysis. The model for the variants is most likely similar to that for the wild type except that the generation of autolysed protein (Ap) has been substantially slowed. Thermal unfolding in the presence and absence of citrate was key to highlighting that active and intact variants in the presence of citrate have a folded structure. This data is suggests that the citrate-stable substitutions have most likely increased the stability of the calcium-stressed protein given that none of the mutations discovered are directly involved in calcium binding. Thus as described herein, citrate-induced instability of NprE due to loss of calcium can be indirectly remediated by improving the stability of the calcium stressed protein.

Specifically, the citrate-induced inactivation and autolysis of the wild-type *Bacillus amyloliquefaciens* NprE protease, studied using activity assays and SDS-PAGE, was rapid and irreversible. The N-termini of the initial autolysis sites identified by Edman degradation, are located in the loop-regions and on the surface of the molecule. Single-site saturation mutagenesis followed by combinatorial mutagenesis resulted in an exemplary citrate-stable protein with five amino acid substitutions at S129, F130, M138, V 190, and D220, which are distributed in loop-and surface-site regions. The S129I-F130L-M138L-V1901-D220P molecule is completely active and structurally intact in the presence of 100 mM citrate when incubated at room temperature overnight. The calorimetric determination of the thermal melting points, in the presence of citrate, for the single and multiple combinatorial variants showed additivity and the apparent $\Delta$Tm is +10 °C. This stabilization effect contemplated to be due to the creation of a stable native state that does not undergo the process of irreversible inactivation and autolysis. As industrial detergents frequently contain citrate, the present invention provides extremely beneficial opportunities for application in the production and development of industrial detergents containing NprE variants.

## Detailed Description of Cleaning and Detergent Formulations of the Present Invention

Unless otherwise noted, all component or composition levels provided herein are made in reference to the active level of that component or composition, and are exclusive of impurities, for example, residual solvents or by-products, which may be present in commercially available sources. Enzyme components weights are based on total active protein. All percentages and ratios are calculated by weight unless otherwise indicated. All percentages and ratios are calculated based on the total composition unless otherwise indicated.

In the exemplified detergent compositions, the enzymes levels are expressed by pure enzyme by weight of the total composition and unless otherwise specified, the detergent ingredients are expressed by weight of the total compositions.

## Cleaning Compositions Comprising Neutral Metalloprotease

The neutral metalloproteases of the present invention are useful in formulating various detergent compositions. The cleaning composition of the present invention may be advantageously employed for example, in laundry applications, hard surface cleaning, automatic dishwashing applications, as well as cosmetic applications such as dentures, teeth, hair and skin. However, due to the unique advantages of increased effectiveness in lower temperature solutions and the superior color-safety profile, the enzymes of the present invention are ideally suited for laundry applications such as the bleaching of fabrics. Furthermore, the enzymes of the present invention find use in both granular and liquid compositions.

The enzymes of the present invention also find use in cleaning additive products. A cleaning additive product including at least one enzyme of the present invention is ideally suited for inclusion in a wash process when additional bleaching effectiveness is desired. Such instances include, but are not limited to low temperature solution cleaning applications. The additive product may be, in its simplest form, one or more neutral metalloprotease enzyme as provided by the present invention. In some embodiments, the additive is packaged in dosage form for addition to a cleaning process where a source of peroxygen is employed and increased bleaching effectiveness is desired. In some embodiments, the single dosage form comprises a pill, tablet, gelcap or other single dosage unit including pre-measured powders and/or liquids. In some embodiments, filler and/or carrier material(s) are included, in order to increase the volume of such composition. Suitable filler or carrier materials include, but are not limited to, various salts of sulfate, carbonate and silicate as well as talc, clay and the like. In some embodiments filler and/or carrier materials for liquid compositions include water and/or low molecular weight primary and secondary alcohols including polyols and diols. Examples of such alcohols include, but are not limited to, methanol, ethanol, propanol and isopropanol. In some embodiments, the compositions comprise from about 5% to about 90% of such materials. In additional embodiments, acidic fillers are used to reduce the pH of the composition. In some alternative embodiments the cleaning additive includes at least one activated peroxygen source as described below and/or adjunct ingredients as more fully described below.

The cleaning compositions and cleaning additives of the present invention require an effective amount of neutral metalloprotease enzyme as provided in the present invention. In some embodiments, the required level of enzyme is achieved by the addition of one or more species of neutral metalloprotease provided by the present invention. Typically, the cleaning compositions of the present invention comprise at least 0.0001 weight percent, from about 0.0001 to about 1, from about 0.001 to about 0.5, or even from about 0.01 to about 0.1 weight percent of at least one neutral metalloprotease provided by the present invention.

In some preferred embodiments, the cleaning compositions provided herein are typically formulated such that, during use in aqueous cleaning operations, the wash water has a pH of from about 5.0 to about 11.5, or in alternative embodiments, even from about 6.0 to about 10.5. In some preferred embodiments, liquid product formulations are typically formulated to have a neat pH from about 3.0 to about 9.0, while in some alternative embodiments the formulation has a neat pH from about 3 to about 5. In some preferred embodiments, granular laundry products are typically formulated to have a pH from about 8 to about 11. Techniques for controlling pH at recommended usage levels include the use of buffers, alkalis, acids, etc., and are well known to those skilled in the art.

In some particularly preferred embodiments, when at least one neutral metalloprotease is employed in a granular composition or liquid, the neutral metalloprotease is in the form of an encapsulated particle to protect the enzyme from other components of the granular composition during storage. In addition, encapsulation also provides a means of controlling the availability of the neutral metalloprotease(s) during the cleaning process and may enhance performance of the neutral metalloprotease(s). It is contemplated that the encapsulated neutral metalloproteases of the present invention will find use in various settings. It is also intended that the neutral metalloprotease be encapsulated using any suitable encapsulating material(s) and method(s) known in the art.

In some preferred embodiments, the encapsulating material typically encapsulates at least part of the neutral metalloprotease catalyst. In some embodiments, the encapsulating material is water-soluble and/or water-dispersible. In some additional embodiments, the encapsulating material has a glass transition temperature (Tg) of 0°C or higher (*See e.g.,* WO 97/11151, particularly from page 6, line 25 to page 7, line 2, for more information regarding glass transition temperatures).

In some embodiments, the encapsulating material is selected from the group consisting of carbohydrates, natural or synthetic gums, chitin and chitosan, cellulose and cellulose derivatives, silicates, phosphates, borates, polyvinyl alcohol, polyethylene glycol, paraffin waxes and combinations thereof. In some embodiments in which the encapsulating material is a carbohydrate, it is selected from the group consisting of monosaccharides, oligosaccharides, polysaccharides, and combinations thereof. IN some preferred embodiments, the encapsulating material is a starch (*See e.g.,* EP 0 922 499; US 4,977,252. US 5,354,559, and US 5,935,826, for descriptions of some exemplary suitable starches).

In additional embodiments, the encapsulating material comprises a microsphere made from plastic(*e.g.*, thermoplastics, acrylonitrile, methacrylonitrile, polyacrylonitrile, polymethacrylonitrile and mixtures thereof; commercially available microspheres that find use include, but are not limited to EXPANCEL® [Casco Products, Stockholm, Sweden], PM 6545, PM 6550, PM 7220, PM 7228, EXTENDOSPHERES®, and Q-CEL® [PQ Corp., Valley Forge, PA], LUXSIL® and SPHERICELI® [Potters Industries, Inc., Carlstadt, NJ and Valley Forge, PA]).

## Processes of Making and Using of Applicants' Cleaning Composition

In some preferred embodiments compositions of the present invention are formulated into any suitable form and prepared by any process chosen by the formulator, *(See e.g.,* U.S. 5.879,584, U.S. 5,691,297, U.S. 5,574,005, U.S. 5,569,645, U.S. 5,565,422, U.S. 5,516,448, U.S. 5,489,392, and U.S. 5,486,303, for some non-limiting examples). In some embodiments in which a low pH cleaning composition is desired, the pH of such composition is adjusted via the addition of an acidic material such as HCl.

## Adjunct Materials

While not essential for the purposes of the present invention, in some embodiments, the non-limiting list of adjuncts described herein are suitable for use in the cleaning compositions of the present invention. Indeed, in some embodiments, adjuncts are incorporated into the cleaning compositions of the present invention. In some embodiments, adjunct materials assist and/or enhance cleaning performance, treat the substrate to be cleaned, and/or modify the aesthetics of the cleaning composition (*e.g..* perfumes, colorants, dyes, etc.). It is understood that such adjuncts are in addition to the neutral metalloproteases of the present invention. The precise nature of these additional components, and levels of incorporation thereof, depends on the physical form of the composition and the nature of the cleaning operation for which it is to be used. Suitable adjunct materials include, but are not limited to, surfactants, builders, chelating agents, dye transfer inhibiting agents, deposition aids, dispersants, additional enzymes, and enzyme stabilizers, catalytic materials, bleach activators, bleach boosters, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, processing aids and/or pigments. In addition to those provided explicitly herein, additional examples are known in the art (*See e.g.,* U.S. Patent Nos. 5,576,282, 6,306,812 B1 and 6,326,348 B1). In some embodiments, the aforementioned adjunct ingredients constitute the balance of the cleaning compositions of the present invention.

**Surfactants -** In some embodiments, the cleaning compositions of the present invention comprise at least one surfactant or surfactant system, wherein the surfactant is selected from nonionic surfactants, anionic surfactants, cationic surfactants, ampholytic surfactants, zwitterionic surfactants, semi-polar nonionic surfactants, and mixtures thereof. In some low pH cleaning composition embodiments (e.g., compositions having a neat pH of from about 3 to about 5), the composition typically does not contain alkyl ethoxylated sulfate, as it is believed that such surfactant may be hydrolyzed by such compositions the acidic contents.

In some embodiments, the surfactant is present at a level of from about 0.1% to about 60%, while in alternative embodiments, the level is from about 1% to about 50% , while in still further embodiments, the level is from about 5% to about 40%, by weight of the cleaning composition.

**Builders -** In some embodiments, the cleaning compositions of the present invention comprise one or more detergent builders or builder systems. In some embodiments incorporating at least one builder, the cleaning compositions comprise at least about 1%, from about 3% to about 60% or even from about 5% to about 40% builder by weight of the cleaning composition.

Builders include, but are not limited to, the alkali metal, ammonium and alkanolammonium salts of polyphosphates, alkali metal silicates, alkaline earth and alkali metal carbonates, aluminosilicate builders polycarboxylate compounds, ether hydroxypolycarboxylates, copolymers of maleic anhydride with ethylene or vinyl methyl ether, 1, 3, 5-trihydroxy benzene-2, 4, 6-trisulphonic acid, and carboxymethyloxysuccinic acid, the various alkali metal, ammonium and substituted ammonium salts of polyacetic acids such as ethylenediamine tetraacetic acid and nitrilotriacetic acid, as well as polycarboxylates such as mellitic acid, succinic acid, citric acid, oxydisuccinic acid, polymaleic acid, benzene 1,3,5-tricarboxylic acid, carboxymethyloxysuccinic acid, and soluble salts thereof. Indeed, it is contemplated that any suitable builder will find use in various embodiments of the present invention.

**Chelating Agents -** In some embodiments, the cleaning compositions of the present invention contain at least one chelating agent, Suitable chelating agents include, but are not limited to copper, iron and/or manganese chelating agents and mixtures thereof. In embodiments in which at least one chelating agent is used, the cleaning compositions of the present invention comprise from about 0.1% to about 15% or even from about 3.0% to about 10% chelating agent by weight of the subject cleaning composition.

**Deposition Aid -** In some embodiments, the cleaning compositions of the present invention include at least one deposition aid. Suitable deposition aids include, but are not limited to polyethylene glycol, polypropylene glycol, polycarboxylate, soil release polymers such as polytelephthalic acid, clays such as kaolinite, montmorillonite, atapulgite, illite, bentonite, halloysite, and mixtures thereof.

**Dye Transfer Inhibiting Agents -** In some embodiments, the cleaning compositions of the present invention include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof.

In embodiments in which at least one dye transfer inhibiting agent is used, the cleaning compositions of the present invention comprise from about 0.0001% to about 10%, from about 0.01 % to about 5%, or even from about 0.1% to about 3% by weight of the cleaning composition.

**Dispersants -** In some embodiments, the cleaning compositions of the present invention contains at least one dispersants. Suitable water-soluble organic materials include, but are not limited to the homo- or co-polymeric acids or their salts, in which the polycarboxylic acid comprises at least two carboxyl radicals separated from each other by not more than two carbon atoms.

**Enzymes -** In some embodiments, the cleaning compositions of the present invention comprise one or more detergent enzymes which provide cleaning performance and/or fabric care benefits. Examples of suitable enzymes include, but are not limited to, hemicellulases, peroxidases, proteases, cellulases, xylanases, lipases, phospholipases, esterases, cutinases, pectinases, keratinases, reductases, oxidases, phenoloxidases, lipoxygenases, ligninases, pullulanases, tannases, pentosanases, malanases, ß-glucanases, arabinosidases, hyaluronidase, chondroitinase, laccase, and amylases, or mixtures thereof. In some embodiments, a combination of enzymes is used (*i.e.,* a "cocktail") comprising conventional applicable enzymes like protease, lipase, cutinase and/or cellulase in conjunction with amylase is used.

<u>Enzyme Stabilizers</u> - In some embodiments of the present invention, the enzymes used in the detergent formulations of the present invention are stabilized. It is contemplated that various techniques for enzyme stabilization will find use in the present invention. For example, in some embodiments, the enzymes employed herein are stabilized by the presence of water-soluble sources of zinc (II), calcium (II) and/or magnesium (II) ions in the finished compositions that provide such ions to the enzymes, as well as, other metal ions (*e.g.,* barium (II), scandium (II), iron (II), manganese (II), aluminum (III), Tin (II), cobalt (II), copper (II), Nickel (II), and oxovanadium (IV)).

<u>Catalytic Metal Complexes</u> -- In some embodiments, the cleaning compositions of the present invention contain one or more catalytic metal complexes. In some embodiments, a metal-containing bleach catalyst finds use. In some preferred embodiments, the metal bleach catalyst comprises a catalyst system comprising a transition metal cation of defined bleach catalytic activity, (*e.g.*, copper, iron, titanium, ruthenium, tungsten, molybdenum, or manganese cations), an auxiliary metal cation having little or no bleach catalytic activity (*e.g.*, zinc or aluminum cations), and a sequestrate having defined stability constants for the catalytic and auxiliary metal cations, particularly ethylenediaminetetraacetic acid, ethylenediaminetetra (methylenephosphonic acid) and water-soluble salts thereof are used (*See e.g.,* U.S. 4,430,243).

In some embodiments, the cleaning compositions of the present invention are catalyzed by means of a manganese compound. Such compounds and levels of use are well known in the art *(See e.g.,* U.S. 5,576,282).

In additional embodiments, cobalt bleach catalysts find use in the cleaning compositions of the present invention. Various cobalt bleach catalysts are known in the art (*See e.g.,* U.S. 5.597.936. and U.S. 5.595.967). Such cobalt catalysts are readily prepared by known procedures (*See e.g.* U.S. 5.597.936. and U.S. 5.595.967).

In additional embodiments, the cleaning compositions of the present invention include a transition metal complex of a macropolycyclic rigid ligand ("MRL"). As a practical matter, and not by way of limitation, in some embodiments, the compositions and cleaning processes provided by the present invention are adjusted to provide on the order of at least one part per hundred million of the active MRL species in the aqueous washing medium, and in some preferred embodiments, provide from about 0.005 ppm to about 25 ppm, more preferably from about 0.05 ppm to about 10 ppm, and most preferably from about 0.1 ppm to about 5 ppm, of the MRL in the wash liquor.

Preferred transition-metals in the instant transition-metal bleach catalyst include, but are not limited to manganese, iron and chromium. Preferred MRLs also include, but are not limited to special ultra-rigid ligands that are cross-bridged (*e.g.*, 5.12-diethyl-1,5,8.12-tetraazabicyclo[6.6.2]hexadecane). Suitable transition metal MRLs are readily prepared by known procedures *(See e.g.,* WO 00/32601, and U.S. 6,225,464).

## Processes of Making and Using Cleaning Compositions

The cleaning compositions of the present invention are formulated into any suitable form and prepared by any suitable process chosen by the formulator, (*See e.g.,* U.S. 5,879,584, U.S. 5,691,297, U.S. 5.574,005. U.S. 5,569,645, U.S. 5,565,422, U.S. 5,516,448. U.S. 5,489,392. U.S. 5,486,303. U.S. 4.515.705. U.S. 4,537,706. U.S. 4,515,707. U.S. 4,550,862. U.S. 4,561,998. U.S. 4,597,898. U.S. 4,968,451. U.S. 5,565,145. U.S. 5,929,022. U.S. 6,294,514. and U.S. 6.376.4451.

## Method of Use

In preferred embodiments, the cleaning compositions of the present invention find use in cleaning surfaces and/or fabrics. In some embodiments, at least a portion of the surface and/or fabric is contacted with at least one embodiment of the cleaning compositions of the present invention, in neat form or diluted in a wash liquor, and then the surface and/or fabric is optionally washed and/or rinsed. For purposes of the present invention. "washing" includes, but is not limited to scrubbing, and mechanical agitation, In some embodiments, the fabric comprises any fabric capable of being laundered in normal consumer use conditions. In preferred embodiments, the cleaning compositions of the present invention are used at concentrations of from about 500 ppm to about 15.000 ppm in solution. In some embodiments in which the wash solvent is water. the water temperature typically ranges from about 5 °C to about 90°C. In some preferred embodiments for fabric cleaning, the water to fabric mass ratio is typically from about 1:1 to about 30: I.

## EXPERIMENTAL

The following examples are provided in order to demonstrate and further illustrate certain preferred embodiments and aspects of the present invention and are not to be construed as limiting the scope thereof.

Some of the following illustrate the invention as defined in the claims; others provide useful background.

In the experimental disclosure which follows, the following abbreviations apply: °C (degrees Centigrade); rpm (revolutions per minute); H$_2$O (water); HCl (hydrochloric acid); aa and AA (amino acid); bp (base pair); kb (kilobase pair); kD (kilodaltons); gm (grams); $\mu$g and ug (micrograms); mg (milligrams); ng (nanograms); $\mu$l and ul (microliters); ml (milliliters); mm (millimeters); nm (nanometers); $\mu$m and $\mu$m (micrometer); M (molar); mM (millimolar); $\mu$M and uM (micromolar); U (units); V (volts); MW (molecular weight); sec (seconds); min(s) (minute/minutes); hr(s) (hour/hours); $MgCl_2$ (magnesium chloride); NaCl (sodium chloride); $OD_{280}$ (optical density at 280 nm); $OD_{405}$ (optical density at 405 nm); $OD_{600}$ (optical density at 600 nm); PAGE (polyacrylamide gel electrophoresis); EtOH (ethanol); PBS (phosphate buffered saline [150 mM NaCl. 10 mM sodium phosphate buffer, pH 7.2]); LAS (lauryl sodium sulfonate); SDS (sodium dodecyl sulfate); Tris (tris(hydroxymethyl)aminomethane); TAED (N,N,N'N'-tetraacetylethylenediamine); BES (polyesstersulfone); MES (2-inorpholinoethanesulfonic acid, monohydrate; f.w. 195.24; Sigma # M-3671); $CaCl_2$ (calcium chloride, anhydrous; f.w. 110.99; Sigma # C-4901); DMF (N,N-dimethylformamide, f.w. 73.09, d = 0.95); Abz-AGLA-Nba (2-aminobenzoyl-L-alanyl-glycyl-L-leucyl-L-alanino-4-nitrobenzylamide. f.w. 583.65; Bachem # H-6675. VWR catalog # 100040-598); SBG1% (Super Broth with Glucose; 6 g Soytone [Difco], 3 g yeast extract, 6 g NaCl. 6 g glucose); the pH was adjusted to 7.1 with NaOH prior to sterilization using methods known in the art; w/v (weight to volume); v/v (volume to volume); Npr and npr (neutral metalloprotease); SEQUEST® (SEQUEST database search program. University of Washington); MS (mass spectroscopy); BMI (blood. milk. ink); SRI (Stain Removal Index); *Npr* and *npr* (neutral metalloprotease gene); NprE and nprE *(B. amyloliquefaciens* neutral metalloprotease); PMN (purified MULTIFECT® metalloprotease); and Quint (S129I-F130L-M138L-V 1901-D220P quintuple NprE variant).

The following abbreviations apply to companies whose products or services may have been referred to in the experimental examples: TIGR (The Institute for Genomic Research. Rockville, MD); AATCC (American Association of Textile and Coloring Chemists): Amersham (Amersham Life Science, Inc. Arlington Heights, IL); Corning (Corning International, Corning, NY); ICN (ICN Pharmaceuticals, Inc., Costa Mesa, CA); Pierce (Pierce Biotechnology, Rockford, IL); Equest (Equest, Warwick International Group, Inc., Flintshire, UK); EMPA (Eidgenossische Material Prufungs und Versuch Anstalt, St. Gallen, Switzerland); CFT (Center for Test Materials, Vlaardingen, The Netherlands); Amicon (Amicon, Inc., Beverly, MA); ATCC (American Type Culture Collection, Manassas, VA); Becton Dickinson (Becton Dickinson Labware, Lincoln Park, NJ); Perkin-Elmer (Perkin-Elmer, Wellesley, MA); Rainin (Rainin Instrument, LLC. Woburn, MA); Eppendorf (Eppendorf AG, Hamburg, Germany); Waters (Waters, Inc., Milford, MA); Geneart (Geneart GmbH, Regensburg, Germany); Perseptive Biosystems (Perseptive Biosystems, Ramsey, MN); Molecular Probes (Molecular Probes, Eugene, OR); BioRad (BioRad, Richmond, CA); Clontech (CLONTECH Laboratories, Palo Alto, CA); Cargill (Cargill, Inc., Minneapolis, MN); Difco (Difco Laboratories, Detroit, MI); GIBCO BRL or Gibco BRL (Life Technologies, Inc., Gaithersburg, MD); New Brunswick (New Brunswick Scientific Company, Inc., Edison, NJ); Thermoelectron (Thermoelectron Corp., Waltham, MA); BMG (BMG Labtech, GmbH, Offenburg, Germany);Greiner (Greiner Bio-One, Kremsuenster, Austria); Novagen (Novagen, Inc., Madison, WI); Novex (Novex, San Diego, CA); Finnzymes (Finnzymes OY, Finland) Qiagen (Qiagen, Inc., Valencia, CA); Invitrogen (Invitrogen Corp., Carlsbad, CA); Sigma (Sigma Chemical Co., St. Louis, MO); DuPont Instruments (Asheville, NY); Global Medical Instrumentation or GMI (Global Medical Instrumentation; Ramsey, MN); MJ Research (MJ Research, Waltham, MA); Infors (Infors AG, Bottmingen, Switzerland); Stratagene (Stratagene Cloning Systems, La Jolla, CA); Roche (Hoffmann La Roche, Inc., Nutley, NJ); Agilent (Agilent Technologies, Palo Alto, CA); S-Matrix (S-Matrix Corp., Eureka, CA); US Testing (United States Testing Co., Hoboken, NY); West Coast Analytical Services (West Coast Analytical Services, Inc., Santa Fe Springs, CA); Ion Beam Analysis Laboratory (Ion Bean Analysis Laboratory, The University of Surrey Ion Beam Centre (Guildford, UK); TOM (Terg-o-Meter); BaChem (BaChem AG, Bubendorf, Switzerland); Molecular Devices (Molecular Devices, Inc., Sunnyvale, CA); Coming (Corning International, Coming, NY); MicroCal (Microcal. Inc., Northhampton, MA); Chemical Computing (Chemical Computing Corp., Montreal, Canada); NCBI (National Center for Biotechnology Information); Argo Bioanalytica (Argo Bioanalytica. Inc, New Jersey); Vydac (Grace Vydac, Hesperia, CA); Minolta (Konica Minolta, Ramsey, NJ); and Zeiss (Carl Zeiss, Inc., Thornwood, NY).

The following sequences find use in and are provided by the present invention.

SEQ ID NO:1 (NprE)
GTGGGTTTAGGTAAGAAATTGTCTGTTGCTGTCGCCGCTTCCTTTATGAGTTTAACCA
TCAGTCTGCCGGGTGTTCAGGCCGCTGAGAATCCTCAGCTTAAAGAAAACCTGACGA
ATTTTGTACCGAAGCATTCTTTGGTGCAATCAGAATTGCCTTCTGTCAGTGACAAAGC
TATCAAGCAATACTTGAAACAAAACGGCAAAGTCTTTAAAGGCAATCCTTCTGAAAG
ATTGAAGCTGATTGACCAAACGACCGATGATCTCGGCTACAAGCACTTCCGTTATGT
GCCTGTCGTAAACGGTGTGCCTGTGAAAGACTCTCAAGTCATTATTCACGTCGATAA
ATCCAACAACGTCTATGCGATTAACGGTGAATTAAACAACGATGTTTCCGCCAAAAC
GGCAAACAGCAAAAAATTATCTGCAAATCAGGCGCTGGATCATGCTTATAAAGCGAT
CGGCAAATCACCTGAAGCCGTTTCTAACGGAACCGTTGCAAACAAAAACAAAGCCG
AGCTGAAAGCAGCAGCCACAAAAGACGGCAAATACCGCCTCGCCTATGATGTAACC
ATCCGCTACATCGAACCGGAACCTGCAAACTGGGAAGTAACCGTTGATGCGGAAAC
AGGAAAAATCCTGAAAAAGCAAAACAAAGTGGAGCATGCCGCCACAACCGGAACA
GGTACGACTCTTAAAGGAAAAACGGTCTCATTAAATATTTCTTCTGAAAGCGGCAAA
TATGTGCTGCGCGATCTTTCTAAACCTACCGGAACACAAATTATTACGTACGATCTGC
AAAACCGCGAGTATAACCTGCCGGGCACACTCGTATCCAGCACCACAAACCAGTTTA
CAACTTCTTCTCAGCGCGCTGCCGTTGATGCGCATTACAACCTCGGCAAAGTGTATG
ATTATTTCTATCAGAAGTTTAATCGCAACAGCTACGACAATAAAGGCGGCAAGATCG
TATCCTCCGTTCATTACGGCAGCAGATACAATAACGCAGCCTGGATCGGCGACCAAA
TGATTTACGGTGACGGCGACGGTTCATTCTTCTCACCTCTTTCCGGTTCAATGGACGT
AACCGCTCATGAAATGACACATGGCGTTACACAGGAAACAGCCAACCTGAACTACG
AAAATCAGCCGGGCGCTTTAAACGAATCCTTCTCTGATGTATTCGGGTACTTCAACG
ATACTGAGGACTGGGATATCGGTGAAGATATTACGGTCAGCCAGCCGGCTCTCCGCA
GCTTATCCAATCCGACAAAATACGGACAGCCTGATAATTTCAAAAATTACAAAAACC
TTCCGAACACTGATGCCGGCGACTACGGCGGCGTGCATACAAACAGCGGAATCCCG
AACAAAGCCGCTTACAATACGATTACAAAAATCGGCGTGAACAAAGCGGAGCAGAT
TTACTATCGTGCTCTGACGGTATACCTCACTCCGTCATCAACTTTTAAAGATGCAAAA
GCCGCTTTGATTCAATCTGCGCGGGACCTTTACGGCTCTCAAGATGCTGCAAGCGTA
GAAGCTGCCTGGAATGCAGTCGGATTGTAA


SEQ ID NO:2 (NprE precursor)
MGLGKKLSVAVAASFMSLTISLPGVQAAENPQLKENLTNFVPKHSLVQSELPSVSDKAIK
QYLKQNGKVFKGNPSERLKLIDQTTDDLGYKHFRYVPVVNGVPVKDSQVIIHVDKSNNV
YAINGELNNDVSAKTANSKKLSANQALDHAYKAIGKSPEAVSNGTVANKNKAELKAAA
TKDGKYRLAYDVTIRYIEPEPANWEVTVDAETGKILKKQNKVEHAATTGTGTTLKGKTV
SLNISSESGKYVLRDLSKPTGTQIITYDLQNREYNLPGTLVSSTTNQFTTSSQRAAVDAHY
NLGKVYDYFYQKFNRNSYDNKGGKIVSSVHYGSRYNNAAWIGDQMIYGDGDGSFFSPL
SGSMDVTAHEMTHGVTQETANLNYENQPGALNESFSDVFGYFNDTEDWDIGEDITVSQP
ALRSLSNPTKYGQPDNFKNYKNLPNTDAGDYGGVHTNSGIPNKAAYNTITKIGVNKAEQ
IYYRALTVYLTPSSTFKDAKAALIQSARDLYGSQDAASVEAAWNAVGL


SEQ ID NO:3 (NprE mature)
AATTGTGTTLKGKTVSLNISSESGKYVLRDLSKPTGTQIITYDLQNREYNLPGTLVSSTTN
QFTTSSQRAAVDAHYNLGKVYDYFYQKFNRNSYDNKGGKIVSSVHYGSRYNNAAWIG
DQMIYGDGDGSFFSPLSGSMDVTAHEMTHGVTQETANLNYENQPGALNESFSDVFGYF
NDTEDWDIGEDITVSQPALRSLSNPTKYGQPDNFKNYKNLPNTDAGDYGGVHTNSGIPN
KAAYNTITKIGVNKAEQIYYRALTVYLTPSSTFKDAKAALIQSARDLYGSQDAASVEAA
WNAVGL

SEQ ID NO:4 (NprE primer)

CTGCAGGAATTCAGATCTTAACATTTTTCCCCTATCATTTTTCCCG

SEQ ID NO:5 (NprE primer)
GGATCCAAGCTTCCCGGGAAAAGACATATATGATCATGGTGAAGCC

SEQ ID NO:6 (pUB-BglII-FW)
GTCAGTCAGATCTTCCTTCAGGTTATGACC

SEQ ID NO:7 (pUB-BglII-RV)
GTCTCGAAGATCTGATTGCTTAACTGCTTC

SEQ ID NO:8 (NprE)
AATTGTGTTL

SEQ ID NO:9 (NprE)
DAGDYGGVHT

SEQ ID NO:10 (NprE)
AGDYGGVHTN

SEQ ID NO:11 (NprE)
GDYGGVHTN

SEQ ID NO:12 (NprE)
LSNPTKYGQP

SEQ ID NO:13 (NprE Fragment 1)
**AATTGTGTTL**TVSLNISSESGKYVLRDLSKPTGTQIITYDLQNREYNLPGTLVSSTTNQFT
TSSQRAAVDAHYNLGKVYDYFYQKFNIVSSVHYGSRSLSNPTKYGQPDNFK

SEQ ID NO:14 (NprE Fragment 2)
**DAGDYGGVHT**AAYNTITKAEQIYYRALTVYLTPSSTFKDAKAALIQSARDLYGSQDAAS
VEAAWNAVGL

SEQ ID NO:15 (NprE Fragment 3)
**AATTGTGTTL**TVSLNISSESGKDLSKPTGTQIITYDLQNREYNLPGTLVSSTTNQFTTSSQ
RAAVDAHYNLGKNSYDNKIVSSVHYGSRYNNAAWIGDQMIYGDGDGSFFSPLSGSMD

SEQ ID NO:16 (NprE Fragment 4)
**AATTGTGTTL**TVSLNISSESGKDLSKPTGTQIITYDLQNREYNLPGTLVSSTTNQFTTSSQ
RAAVDAHYNLGKNSYDNKIVSSVHYGSR**MDV**

SEQ ID NO:17 (NprE Fragment 5)
**LSNPTKYGQP**KNYKNLPNTDAGDYGGVHTNSGIPNKAEQIYYRALTVTFKDAKAALIQ
SARDLYGSQDAASVEAAWNAVGL

SEQ ID NO:18 (NprE S129I/F130L/D220P)
AATTGTGTTLKGKTVSLNISSESGKYVLRDLSKPTGTQIITYDLQNREYN
LPGTLVSSTTNQFTTSSQRAAVDAHYNLGKVYDYFYQKFNRNSYDNKGGK
IVSSVHYGSRYNNAAWIGDQMIYGDGDG**IL**FSPLSGSMDVTAHEMTHGVT
QETANLNYENQPGALNESFSDVFGYFNDTEDWDIGEDITVSQPALRSLSN

PTKYGQPDNFKNYKNLPNTPAGDYGGVHTNSGIPNKAAYNTITKIGVNKA
EQIYYRALTVYLTPSSTFKDAKAALIQSARDLYGSQDAASVEAAWNAVGL

SEQ ID NO:19 (NprE M138L/V190I/D220P)
AATTGTGTTLKGKTVSLNISSESGKYVLRDLSKPTGTQIITYDLQNREYNLPGTLVSSTT
NQFTTSSQRAAVDAHYNLGKVYDYFYQKFNRNSYDNKGGKIVSSVHYGSRYNNAAWI
GDQMIYGDGDGSFFSPLSGSLDVTAHEMTHGVTQETANLNYENQPGALNESFSDVFGYF
NDTEDWDIGEDITISQPALRSLSNPTKYGQPDNFKNYKNLPNTPAGDYGGVHTNSGIPNK
AAYNTITKIGVNKAEQIYYRALTVYLTPSSTFKDAKAALIQSARDLYGSQDAASVEAAW
NAVGL

SEQ ID NO:20 (NprE S129I/F130L/M138L/V190I/D220P)
AATTGTGTTLKGKTVSLNISSESGKYVLRDLSKPTGTQIITYDLQNREYN
LPGTLVSSTTNQFTTSSQRAAVDAHYNLGKVYDYFYQKFNRNSYDNKGGK
IVSSVHYGSRYNNAAWIGDQMIYGDGDGILFSPLSGSLDVTAHEMTHGVT
QETANLNYENQPGALNESFS DVFGYFNDTEDWDIGEDITI SQPALRSLSN
PTKYGQPDNFKNYKNLPNTPAGDYGGVHTN SGIPNKAAYNTITKIGVNKA
EQIYYRALTVYLTPSSTFKDAKAALIQSARDLYGSQDAASVEAAWNAVGL

## EXAMPLE 1

**Assays**

The following assays were used in the examples described below. Any deviations from the protocols provided below are indicated in the examples. In these experiments, a spectrophotometer was used to measure the absorbance of the products formed after the completion of the reactions. A reflectometer was used to measure the reflectance of the swatches.

### A. Protein Content Determination

### 1. BCA (bicinchoninic acid) Assay for Protein Content Determination in 96-well Microtiter Plates (MTPs)

In these assays, BCA (Pierce) assay was used to determine the protein concentration in protease samples on MTP scale. In this assay system, the chemical and reagent solutions used were: BCA protein assay reagent, and Pierce Dilution buffer (50 mM MES, pH 6.5, 2mM $CaCl_2$, 0.005% TWEEN®-80). The equipment used was a SpectraMAX (type 340) MTP reader. The MTPs were obtained from Costar (type 9017).

In the test, 200 $\mu$l BCA Reagent was pipetted into each well, followed by 20 $\mu$l diluted protein. After thorough mixing, the MTPs were incubated for 30 minutes at 37°C. Possible air bubbles were removed, and the optical density (OD) of the solution within the wells was read at 562 nm. To determine the protein concentration, the background reading was subtracted form the sample readings. The $OD_{562}$ was plotted for against protein standards (purified protease), to produce a standard curve. The protein concentration of the samples was extrapolated from the standard curve.

### 2. Bradford Assay for Protein Content Determination in 96-well Microtiter Plates (MTPs)

In these assays, the Bradford dye reagent (Quick Start) assay was used to determine the protein concentration in protease samples on MTP scale.

In this assay system, the chemical and reagent solutions used were: Quick Start Bradford Dye Reagent (BIO-RAD Catalog No. 500-0205), Dilution buffer (10mM NaCl, 0.1mM CaCl2, 0.005% TWEEN®-80). The equipment used was a Biomek FX Robot (Beckman) and a SpectraMAX (type 340) MTP reader. The MTPs were from Costar (type 9017).

In the test, 200 $\mu$l Bradford Dye Reagent was pipetted into each well, followed by 15 $\mu$l dilution buffer. Finally 10 $\mu$l of filtered culture broth were added to the wells.

After thorough mixing, the MTPs were incubated for at least 10 minutes at room temperature. Possible air bubbles were blown away and the ODs of the wells were read at 595 nm. To determine the protein concentration, the background reading (*i.e.*, from uninoculated wells) was subtracted form the sample readings. The obtained $OD_{595}$ values provide a

relative measure of the protein content in the samples.

## B. Citrate Stability Assay For Proteolytic Activity

Citrate stability was measured after incubation of wild-type NprE and variants in the presence of mM citrate. The initial and residual activity was determined using the DMC hydrolysis assay. In this assay system, the chemical and reagent solutions used were:

| | |
|---|---|
| Citric acid monohydrate | Merck 1.00244 |
| Pipes (free acid) | Sigma P-1851 |
| Tris (free acid) | Sigma T-1378 |
| HEPES (Ultra>99.5%) | Sigma-H7523 |
| TWEEN®-80 | Sigma P-8074 |
| Dimethylcasein (DMC) | Sigma C-9801 |
| Tris buffer (free acid) | 6.04 g dissolved in 1000 ml water (= 50 mM) |
| HEPES buffer | 11.9 g. dissolved in 1000 ml water (= 50 mM) |
| Citrate buffer (free acid) | 21.0 g. dissolved in 1000 ml water (= 100 mM), |
| PIPES buffer (free acid): | 3.32 g dissolved in about 960 ml water, |
| DMC solution | 1% w/v in 55 mM PIPES buffer, final pH = 6.0 |
| Dilution buffer 1 | 0.1 mM CaC12/25 mM Tris; pH 8.2 |
| Dilution buffer 2 | 0.1 mM CaC12/50 mM Citrate/25 mM Tris; pH8.2 |

The concentrations of these dilution buffers are indicated as final concentrations. The initial concentration was proportionally higher and dependent on the dilution rate. In alternative experiments, HEPES finds use in place of Tris. The equipment used was a Biomek FX Robot (Beckman), and an incubator/shaker (Innova, type 4230; New Brunswick). The PIPES buffer was adjusted to pH 5.8 with 4 N HCl (final concentration of 55 mM). The Tris buffer was adjusted to pH 8.2 with 4 N HCl (final concentration of 25 mM). The 50 mM citrate/25 mM Tris buffer was adjusted to pH 8.2 with 4 N NaOH. The HEPES buffer was adjusted to pH 8.2 with 4 N NaOH (final concentration of 25 mM). The 50 mM citrate/ 25 mM HEPES buffer was adjusted to pH 8.2 with 4 N NaOH.

## Protein Determination and Test Method

In order to establish the desired dilution rate in the citrate stability assay the protease concentration of the wild-type NprE controls for each plate were determined with the TCA assay. In this method. 25 $\mu$l filtered culture broth were added to 200 $\mu$l 16.875% (w/v) TCA. After incubation for 10 to 15 minutes at ambient temperature, the light scattering/ absorbance at 405 nm was determined. The protein concentration was determined by using a calibration line, constructed with purified NprE.

Stressed Conditions:

The filtered culture broth was diluted with dilution buffer 2. The MTP was covered with tape, shaken for a few seconds and placed in the incubator at 25°C for 60 minutes at 200 rpm. After incubation, 20$\mu$l of the mixture were taken from each well and transferred into a new MTP, containing 180 $\mu$l 1% DMC preheated substrate solution (the substrate was preheated at 25°C). The MTP was placed directly in the incubator/shaker and incubated at 25°C for 30 minutes at 200 rpm agitation. The residual protease activity was determined using the dimethylcasein hydrolysis assay, described below.

Unstressed Conditions

The filtered culture broth was diluted with dilution buffer 1. Immediately. 20$\mu$l of the mixture were taken from each well and transferred into a new MTP, containing 180 $\mu$l of preheated 1% DMC substrate solution (the substrate was preheated at 25°C). The MTP was placed directly in the incubator/shaker and incubated for 25°C for 30 minutes at 200 rpm agitation. The initial protease activity as determined with TNBS, using the dimethycasein hydrolysis assay, described below.

All residual activity values (determined with the dimethylcasein hydrolysis assay) were calculated using the following equation.

$$\% \text{ Residual Activity} = \text{OD}_{60\,\text{min}} \text{ value} * 100 / \text{OD}_{00\,\text{min}} \text{ value}$$

## C. Dimethylcasein Hydrolysis Assay

In this assay system, the chemicals and reagent solutions used were:

| | |
|---|---|
| Dimethylcasein (DMC) | Sigma C-9801 |
| TWEEN®-80 | Sigma P-8074 |

PIPES buffer (free acid) Sigma P-1851; 15.1 g dissolved in about 960 ml water; pH adjusted to 6.0 with 4N NaOH, 1 ml of 5% TWEEN®-80 added and the volume brought up to 1000 ml. Final concentration of PIPES and TWEEN®-80: 50 mM and 0.005% respectively.

Picrylsulfonic acid (TNBS) Sigma P-2297 (5% solution in water)

Reagent A   45.4 g $Na_2B_4O_7$.10 $H_2O$ (Merck 6308) and 15 ml of 4N NaOH dissolved together to a final volume of 1000 ml (by heating if needed)

Reagent B   35.2 g $NaH_2PO_4$ $1H_2O$ (Merck 6346) and 0.6 g $Na_2SO_3$ (Merck 6657) dissolved together to a final volume of 1000 ml.

## Method

To prepare the substrate, 4 g dimethylcasein was dissolved in 400 ml PIPES buffer. The filtered culture supernatants were diluted with PIPES buffer. Then, 10 $\mu$l of each diluted supernatant were added to 200 $\mu$l substrate in the wells of a MTP. The MTP was covered with tape, shaken for a few seconds and placed in an oven at 25°C for 30 minutes without agitation. About 15 minutes before removal of the 1st plate from the oven, the TNBS reagent was prepared by mixing 1 ml TNBS solution per 50 ml of Reagent A. MTPs were filled with 60 $\mu$l TNBS Reagent A per well. The incubated plates were shaken for a few seconds, after which 10 $\mu$l was transferred to the MTPs with TNBS Reagent A. The plates were covered with tape and shaken for 20 minutes in a bench shaker (BMG Thermostar) at room temperature and 500 rpm. Finally, 200 $\mu$l Reagent B was added to the wells, mixed for 1 minute on a shaker, and the absorbance at 405 nm was determined using a MTP reader.

The obtained absorbance value was corrected for the blank value (*i.e.*, substrate without enzyme). The resulting absorbance was a measure of the hydrolytic activity. The (arbitrary) specific activity of a sample was calculated by dividing the absorbance and the determined protein concentration.

## D. 2-Aminobenzoyl-L-alanylglycyl-L-leucyl-L-alanino-4-nitrobenzylamide Protease Assay (Abz-AGLA-Nba)

The method provided below provides a degree of technical detail that yields reproducible protease assay data independent of time and place. While the assay can be adapted to a given laboratory condition, any data obtained through a modified procedure must be reconciled with results produced by the original method. Neutral metalloproteases cleave the peptide bond between glycine and leucine of 2-aminobenzoyl-L-alanylglycyl-L-leucyl-L-alanino-4-nitrobenzylamide (Abz-AGLA-Nba). Free 2-aminobenzoyl-L-alanylglycine (Abz-AG) in solution has a fluorescence emission maximum at 415 nm with an excitation maximum of 340 nm. Fluorescence of Abz-AG is quenched by nitrobenzylamide in the intact Abz-AGLA-Nba molecule.

In these experiments, the liberation of Abz-AG by protease cleavage of Abz-AGLA-Nba was monitored by fluorescence spectroscopy (Ex. 340 / Em. 415). The rate of appearance of Abz-AG was a measure of proteolytic activity. Assays were performed under non-substrate limited initial rate conditions.

A microplate mixer with temperature control (*e.g.*, Eppendorf Thermomixer) was required for reproducible assay results. The assay solutions were incubated to desired temperature (*e.g.*, 25°C) in the microplate mixer prior to enzyme addition. Enzyme solutions were added to the plate in the mixer, mixed vigorously and rapidly transferred to the plate reader.

A spectrofluorometer with capability of continuous data recording, linear regression analysis, and temperature control was required (*e.g.*, SpectraMax M5, Gemini EM, Molecular Devices). The reader was always maintained at the desired temperature (*e.g.*, 25°C). The reader was set for top-read fluorescence detection and the excitation was set to 350 nm and emission to 415 nm without the use of a cut-off filter. The PMT was set to medium sensitivity and 5 readings per well. Autocalibration was turned on, but only to calibrate before the first reading. The assay was measured for 3 minutes with the reading interval minimized according to the number of wells being monitored. The reader was set to calculate the rate of milli-RFU/min (thousandths of relative fluorescence units per minute). The number of readings used to calculate the rate (Vmax points) was set to the number equivalent to 2 minutes, as determined by the reading interval (*e.g.*, a reading every 10 seconds would use 12 points to calculate the rate). The max RFU was set to 50,000.

All pipeting of enzyme and substrate stock solutions were done with positive displacement pipets (Rainin Microman). Buffer, assay, and enzyme working solutions were pipetted by single or multi-channel air-displacement pipets (Rainin LTS) from tubes, reagent reservoirs or stock microplates. A repeater pipet (Eppendorf) finds use in transferring the assay solution to microplate wells when few wells are used, to minimize reagent loss. Automated pipetting instruments such as the Beckman FX or Cybio Cybi-well also find use in transferring enzyme solutions from a working stock microplate to the assay microplate in order to initiate an entire microplate at once.

**Reagents and Solutions:**

**52.6 mM MES/NaOH, 2.6 mM CaCl$_2$, pH 6.5 - MES Buffer**

MES acid (10.28 g) and 292 mg anhydrous CaCl$_2$ were dissolved in approximately 900mL purified water. The solution was titrated with NaOH to pH 6.5 (at 25°C or with temperature adjustment pH probe). The pH-adjusted buffer was made up to 1L total volume. The final solution was filtered through a 0.22 μm sterile filter and kept at room temperature.

**48 mM Abz-AGLA-Nba in DMF - Abz-AGLA-Nba Stock**

Approximately 28 mg of Abz-AGLA-Nba was placed in a small tube. It was dissolved in DMF (volume will vary depending upon Abz-AGLA-Nba massed) and vortexed for several minutes. The solution was stored at room temperature shielded from light.

**50 mM MES, 2.5 mM CaCl$_2$, 5% DMF, 2.4 mM Abz-AGLA-Nba pH 6.5 - Assay Solution**

One mL Abz-AGLA-Nba stock was added to 19 mL MES Buffer and vortexed. The solution was stored at room temperature shielded from light.

**50 mM MES, 2.5 mM CaCl$_2$, pH 6.5 - Enzyme Dilution Buffer**

This buffer was produced by adding 5 mL purified water to 95 mL MES Buffer.

**50 mM MES, 2.5 mM CaCl$_2$, 5% DMF, pH 6.5 - Substrate Dilution Buffer**

Five mL pure DMF were added to 95 mL MES Buffer. This buffer was used to determine kinetic parameters.

**Enzyme solutions**

The enzyme stock solutions were diluted with enzyme dilution buffer to a concentration of approximately 1 ppm (1 ug/mL). MULTIFECT® neutral protease (wild-type NprE) was diluted to concentrations below 6 ppm (6 ug/mL). Serial dilutions were preferred. Solutions were stable at room temperature for 1 hour, but for longer term storage, the solutions were maintained on ice.

**Procedure**

First all buffers, stock, and working solutions were prepared. Each enzyme dilution was assayed in triplicate, unless otherwise indicated. When not completely full, the enzyme working solution stock microplate was arranged in full vertical columns starting from the left of the plate (to accommodate the plate reader). The corresponding assay plate was similarly set up. The microplate spectrofluorometer was set up as previously described.

First, a 200 μL aliquot of assay solution were placed in the wells of a 96-well microplate. The plate was incubated for 10 min at 25°C in a temperature controlled microplate mixer, shielded from light. The assay was initiated by transferring 10 uL of the working enzyme solutions from the stock microplate to the assay microplate in the mixer. Optimally, 96-well pipetting head finds use, or an 8-well multi-channel pipet was used to transfer from the left-most column first. The solutions were vigorously mixed for 15 seconds (900rpm in Eppendorf Thermomixer). Immediately, the assay microplate was transferred to the microplate spectrofluorometer and recording of fluorescence measurements at excitation of 350 nm and emission of 415 nm were begun. The spectrofluorometer software calculated the reaction rates of the increase in fluorescence for each well to a linearly regressed line of milli-RFU / min. In some experiments, a second plate was placed in the microplate mixer for temperature equilibration while the first plate was being read.

The rate initial velocities were linear with respect to product concentration (i.e., liberated 2-aminobenzoyl fluorescence) up to 0.3 mM product, which corresponded to approximately 50.000 RFU in a solution starting at 2.3mM Abz-AGLA-Nba with background fluorescence of approximately 22.000 RFU. Abz-AGLA-Nba was dissolved in DMF and used the day it was prepared.

## EXAMPLE 2

### NprE Protease Production in *B. subtilis*

In this Example, experiments conducted to produce NprE protease in *B. subtilis* are described. In particular, the methods used in the transformation of plasmid pUBnprE into B. *subtilis* are provided. Transformation was performed as known in the art *(See e.g.,* WO 2002/014490, and WO 2007/044993.The DNA sequence (nprE leader, nprE pro and nprE mature DNA sequence from *B.amyloliquefaciens*) provided below, encodes the NprE precursor protein:

```
GTGGGTTTAGGTAAGAAATTGTCTGTTGCTGTCGCCGCTTCCTTTATGAGTTTAACCA
TCAGTCTGCCGGGTGTTCAGGCCGCTGAGAATCCTCAGCTTAAAGAAAACCTGACGA
ATTTTGTACCGAAGCATTCTTTGGTGCAATCAGAATTGCCTTCTGTCAGTGACAAAGC
TATCAAGCAATACTTGAAACAAAACGGCAAAGTCTTTAAAGGCAATCCTTCTGAAAG
ATTGAAGCTGATTGACCAAACGACCGATGATCTCGGCTACAAGCACTTCCGTTATGT
GCCTGTCGTAAACGGTGTGCCTGTGAAAGACTCTCAAGTCATTATTCACGTCGATAA
ATCCAACAACGTCTATGCGATTAACGGTGAATTAAACAACGATGTTTCCGCCAAAAC
GGCAAACAGCAAAAAATTATCTGCAAATCAGGCGCTGGATCATGCTTATAAAGCGAT
CGGCAAATCACCTGAAGCCGTTTCTAACGGAACCGTTGCAAACAAAAACAAAGCCG
AGCTGAAAGCAGCAGCCACAAAAGACGGCAAATACCGCCTCGCCTATGATGTAACC
ATCCGCTACATCGAACCGGAACCTGCAAACTGGGAAGTAACCGTTGATGCGGAAAC
AGGAAAAATCCTGAAAAAGCAAAACAAAGTGGAGCATGCCGCCACAACCGGAACA
GGTACGACTCTTAAAGGAAAAACGGTCTCATTAAATATTTCTTCTGAAAGCGGC
AAATATGTGCTGCGCGATCTTTCTAAACCTACCGGAACACAAATTATTACGTAC
GATCTGCAAAACCGCGAGTATAACCTGCCGGGCACACTCGTATCCAGCACCACA
```

AACCAGTTTACAACTTCTTCTCAGCGCGCTGCCGTTGATGCGCATTACAACCTC
GGCAAAGTGTATGATTATTTCTATCAGAAGTTTAATCGCAACAGCTACGACAAT
AAAGGCGGCAAGATCGTATCCTCCGTTCATTACGGCAGCAGATACAATAACGCA
GCCTGGATCGGCGACCAAATGATTTACGGTGACGGCGACGGTTCATTCTTCTCA
CCTCTTTCCGGTTCAATGGACGTAACCGCTCATGAAATGACACATGGCGTTACA
CAGGAAACAGCCAACCTGAACTACGAAAATCAGCCGGGCGCTTTAAACGAATCC
TTCTCTGATGTATTCGGGTACTTCAACGATACTGAGGACTGGGATATCGGTGAA
GATATTACGGTCAGCCAGCCGGCTCTCCGCAGCTTATCCAATCCGACAAAATAC
GGACAGCCTGATAATTTCAAAAATTACAAAAACCTTCCGAACACTGATGCCGGC
GACTACGGCGGCGTGCATACAAACAGCGGAATCCCGAACAAAGCCGCTTACAA
TACGATTACAAAAATCGGCGTGAACAAAGCGGAGCAGATTACTATCGTGCTCT
GACGGTATACCTCACTCCGTCATCAACTTTTAAAGATGCAAAAGCCGCTTTGAT
TCAATCTGCGCGGGACCTTTACGGCTCTCAAGATGCTGCAAGCGTAGAAGCTGC
**CTGGAATGCAGTCGGATTG**TAA (SEQ ID NO:1)

In the above sequence, bold indicates the DNA that encodes the mature NprE protease, standard font indicates the leader sequence (nprE leader), and underlined indicates the pro sequences (nprE pro). The amino acid sequence (NprE leader, NprE pro and NprE mature DNA sequence) provided below (SEQ ID NO:2.), corresponds to the full length NprE precursor protein. In this sequence, underlined indicates the pro sequence and bold indicates the mature NprE protease.

MGLGKKLSVAVAASFMSLTISLPGVQA<u>AENPQLKENLTNFVPKHSLVQSELPSVSDKAIK</u>
<u>QYLKQNGKVFKGNPSERLKLIDQTTDDLGYKHFRYVPVVNGVPVKDSQVIIHVDKSNNV</u>
<u>YAINGELNNDVSAKTANSKKLSANQALDHAYKAIGKSPEAVSNGTVANKNKAELKAAA</u>
<u>TKDGKYRLAYDVTIRYIEPEPANWEVTVDAETGKILKKQNKVEH</u>**AATTGTGTTLKGKT**
**VSLNISSESGKYVLRDLSKPTGTQIITYDLQNREYNLPGTLVSSTTNQFTTSSQRAAV**
**DAHYNLGKVYDYFYQKFNRNSYDNKGGKIVSSVHYGSRYNNAAWIGDQMIYGDGD**
**GSFFSPLSGSMDVTAHEMTHGVTQETANLNYENQPGALNESFSDVFGYFNDTEDWD**
**IGEDITVSQPALRSLSNPTKYGQPDNFKNYKNLPNTDAGDYGGVHTNSGIPNKAAYN**
**TITKIGVNKAEQIYYRALTVYLTPSSTFKDAKAALIQSARDLYGSQDAASVEAAWNA**
**VGL**  (SEQ ID NO:2)

The mature NprE sequence is set forth as SEQ ID NO:3. This sequence was used as the basis for making the variant libraries described herein.

AATTGTGTTLKGKTVSLNISSESGKYVLRDLSKPTGTQIITYDLQNREYNLPGTLVSSTTN

QFTTSSQRAAVDAHYNLGKVYDYFYQKFNRNSYDNKGGKIVSSVHYGSRYNNAAWIG

DQMIYGDGDGSFFSPLSGSMDVTAHEMTHGVTQETANLNYENQPGALNESFSDVFGYF

NDTEDWDIGEDITVSQPALRSLSNPTKYGQPDNFKNYKNLPNTDAGDYGGVHTNSGIPN

KAAYNTITKIGVNKAEQIYYRALTVYLTPSSTFKDAKAALIQSARDLYGSQDAASVEAA

WNAVGL (SEQ ID NO:3)

The pUBnprE expression vector was constructed by amplifying the nprE gene from the chromosomal DNA of *B. amyloliquefaciens* by PCR using two specific primers:

Oligo AB 1740: CTGCAGGAATTCAGATCTTAACATTTTTCCCCTATCATTTTTCCCG (SEQ ID NO:4.)
Oligo AB 1741: GGATCCAAGCTTCCCGGGAAAAGACATATATGATCATGGTGAAGCC (SEQ ID NO:5.)

PCR was performed on a thermocycler with Phusion High Fidelity DNA polymerase (Finnzymes. The PCR mixture contained 10 $\mu$l 5x buffer (Finnzymes Phusion), 1$\mu$l 10mM dNTP's, 1.5$\mu$l DMSO, 1$\mu$l of each primer, 1$\mu$l Finnzymes Phusion DNA polymerase, 1$\mu$l chromosomal DNA solution 50ng/$\mu$l, 34.5 $\mu$l MilliQ water. The following protocol was used:

PCR protocol:

1) 30 sec 98°C;
2) 10 sec 98°C;
3) 20 sec 55°C;
4) 1 min 72°C;
5) 25 cycles of steps 2 to 4; and
6) 5 min 72°C.

This resulted in a 1.9 kb DNA fragment, which was digested using *Bgl*II and *Bcl*I DNA restriction enzymes. The multicopy *Bacillus* vector pUB 110 (*See e.g.,* Gryczan. J Bacteriol, 134:318-329, 1978) was digested with *Bam*HI. The PCR fragment x *Bgl*II x *Bel*I was then ligated in the pUB 110 x *Bam*HI vector to form pUBnprE expression vector.

pUBnprE was transformed to a *B. subtilis* ($\Delta aprE$, $\Delta nprE$, oppA, $\Delta spoIIE$, degUHy32, $\Delta amyE::(xylR,pxylA-comK)$ strain. Transformation into *B. subtilis* was performed as described in WO 02/14490. Selective growth of *B. subtilis* transformants harboring the pUBnprE vector was performed in shake flasks containing 25 ml MBD medium (a MOPS based defined medium), with 20 mg/L neomycin. MBD medium was made essentially as known in the art (*See,* Neidhardt et al.. J Bacteriol. 119: 736-747, 1974), except that $NH_4Cl_2$. $FeSO_4$, and $CaCl_2$ were left out of the base medium, 3 mM $K_2HPO_4$ was used, and the base medium was supplemented with 60 mM urea. 75 g/L glucose, and 1 % soytone. Also, the micronutrients were made up as a 100 X stock containing in one liter, 400 mg $FeSO_4.7H_2O$, 100 mg $MnSO_4.H_2O$, 100 mg $ZnSO_4.7H_2O$, 50 mg $CuCl_2.2H_2O$, 100 mg $CoCl_2.6H_2O$. 100 mg $NaMoO_4.2H_2O$, 100 mg $Na_2B_4O_7.10H_2O$, 10 ml of 1M $CaCl_2$, and 10 ml of 0.5 M sodium citrate. The culture was incubated for three days at 37°C in an incubator/shaker (Infors). This culture resulted in the production of secreted NprE protease with proteolytic activity as demonstrated by protease assays. Gel analysis was performed using NuPage Novex 10% Bis-Tris gels (Invitrogen, Catalog No. NP0301 BOX). To prepare samples for analysis, 2 volumes of supernatant were mixed with I volume 1M HCl. 1 volume 4xLDS sample buffer (Invitrogen, Catalog No. NP0007). and 1% PMSF (20 mg/ml). The samples were subsequently heated for 10 minutes at 70°C. Then, 25 $\mu$L of each sample were loaded onto the gel, together with 10 $\mu$L of SeeBlue plus 2 pre-stained protein standards (Invitrogen, Catalog No. LC5925). The results clearly demonstrated that the nprE cloning strategy described in this example is suitable for production of active NprE in *B. subtilis.*

**EXAMPLE 3**

**Generation of Site Evaluation Libraries (SELs)**

In this Example, methods used in the construction of *nprE* SELs are described.

**Generation of *nprE* SELs - Method I**

The pUBnprE vector, containing the nprE expression cassette described above, served as template DNA. This vector contains a unique *Bgl*II restriction site, which was utilized in the site evaluation library construction. Briefly, to construct a nprE site evaluation library, three PCR reactions were performed, including two mutagenesis PCRs to introduce the mutated codon of interest in the mature nprE DNA sequence and a third PCR used to fuse the two mutagenesis PCRs in order to construct the pUBnprE expression vector including the desired mutated codon in the mature nprE sequence.

The method of mutagenesis was based on the codon-specific mutation approach, in which the creation of all possible mutations at a time in a specific DNA triplet was performed using a forward and reverse oligonucleotide primer with a length of 25 to 45 nucleotides enclosing a specific designed triple DNA sequence NNS (N = A. C. T or G; and S = C or G) that corresponded with the sequence of the codon to be mutated and guaranteed random incorporation of nucleotides at that specific nprE mature codon. The number listed in the primer names corresponds with the specific nprE mature codon position. Multiple sites were evaluated included. An exemplary listing of primer sequences is described in WO 2007/044993.

Two additional primers used to construct the site evaluation libraries contained the *Bgl*II restriction site together with a part of the pUBnprE DNA sequence flanking the *Bgl*II restriction site. These primers were produced by Invitrogen (50 nmole scale, desalted):

pUB-BgIII-FW GTCAGTCAGATCTTCCTTCAGGTTATGACC (SEQ ID NO:6); and
pUB-BgIII-RV GTCTCGAAGATCTGATTGCTTAACTGCTTC (SEQ ID NO:7).

Construction of each SEL started with two primary PCR amplifications using the pUB-BgIII-FW primer and a specific nprE reverse mutagenesis primer. For the second PCR, the pUB-BgIII -RV primer and a specific nprE forward mutagenesis primer (equal nprE mature codon positions for the forward and reverse mutagenesis primers) were used.

The introduction of the mutations in the mature nprE sequence was performed using Phusion High-Fidelity DNA Polymerase (Finnzymes; Catalog No. F-530L). All PCRs were performed according to the Finnzymes protocol supplied with the polymerase. The PCR conditions for the primary PCRs were:

For primary PCR 1:

pUB-BgIII-FW primer and a specific NPRE reverse mutagenesis primer - both 1 μL (10 μM):

For primary PCR 2:

pUB-BgIII -RV primer and a specific NPRE forward mutagenesis primer - both 1 μL (10 μM); together with

| | |
|---|---|
| 5 x Phusion HF buffer | 10 μL |
| 10 mM dNTP mixture | 1 μL |
| Phusion DNA polymerase | 0.75 μL (2 units/ μL) |
| DMSO, 100% | 1 μL |
| pUBnprE template DNA | 1 μL (0.1 - 1 ng/μL) |
| Distilled, autoclaved water | up to 50 μL |

The PCR program was: 30 seconds 98°C. 30x (10 seconds 98°C. 20 seconds 55°C. 1.5 minute 72°C) and 5 min 72°C, performed in a PTC-200 Peltier thermal cycle (MJ Research). The PCR experiments result in two fragments of approximately 2 to 3 kB. which had about 30 nucleotide base overlap around the NprE mature codon of interest. Fragments were fused in a third PCR reaction using these two aforementioned fragments and the forward and reverse *Bgl*II primers. The fusion PCR reaction was carried out in the following solution:

pUB-BgIII-FW primer and pUB-BgIII-RV primer - both 1 μL (10 μM)

together with

| | |
|---|---|
| 5 x Phusion HF buffer | 10 μL |
| 10 mM dNTP mixture | 1 μL |

(continued)

| | |
|---|---|
| Phusion DNA polymerase | 0.75 $\mu$L (2 units/ $\mu$L) |
| DMSO, 100% | 1 $\mu$L |
| primary PCR 1 reaction mix | 1 $\mu$L |
| primary PCR 2 reaction mix | 1 $\mu$L |
| Distilled, autoclaved water | up to 50 $\mu$L |

The PCR fusion program was as follows: 30 seconds 98°C, 30x (10 seconds 98°C. 20 seconds 55°C, 2:40 minute 72°C) and 5 min 72°C, in a PTC-200 Peltier thermal cycler (MJ Research).

The amplified linear 6.5 Kb fragment was purified using the Qiaquick PCR purification kit (Qiagen. Catalog No. 28106) and digested with *Bgl*II restriction enzyme to create cohesive ends on both sides of the fusion fragment:

- 35 $\mu$L purified linear DNA fragment
- 4 $\mu$L REACT® 3 buffer (Invitrogen)
- 1 $\mu$L *Bgl*II. 10 units/ml (Invitrogen)

Reaction conditions: 1 hour, 30°C.

Ligation of the *Bgl*II digested and purified using Qiaquick PCR purification kit (Qiagen, Catalog No. 28106) fragment results in circular and multimeric DNA containing the desired mutation:

- 30 $\mu$L of purified *Bgl*II digested DNA fragment
- 8 $\mu$L T4 DNA Ligase buffer (Invitrogen Catalog No. 46300-018)
- 1 $\mu$L T4 DNA Ligase, 1 unit/$\mu$L (Invitrogen Catalog No. 15224-017)

Reaction conditions: 16-20 hours, 16°C.

Subsequently, the ligation mixture was transformed to a *B. subtilis (ΔaprE, ΔnprE, oppA, ΔspoIIE, degUHy32, ΔamyE::(xylR,pxylA-comK)* strain. Transformation to *B. subtilis* was performed as described in WO 02/14490, incorporated herein by reference. For each library. 96 single colonies were picked and grown in MOPS media with neomycin and 1.25 g/L yeast extract for sequence analysis (BaseClear) and screening purposes. Each library included a maximum of 19 nprE site-specific variants.

The variants were produced by growing the *B. subtilis* SEL transformants in 96 well MTP at 37°C for 68 hours in MBD medium with 20 mg/L neomycin and 1.25 g/L yeast extract.

**Generation of *nprE* SELs - Method II**

Alternative methods to generate *nprE* SELs are also described. These methods are suitable for production of SELs of other enzymes of interest. As above, the pUBnprE vector containing the nprE expression cassette, served as the template DNA source for the generation of *nprE* SELs and NprE variants. The major difference between the two methods is that this method requires amplification of the entire vector using complementary site-directed mutagenic primers.

**Materials:**

*Bacillus* strain containing the pUBnprE vector
Qiagen Plasmid Midi Kit (Qiagen Catalog No. 12143)
Ready-Lyse Lysozyme (Epicentre Catalog No. R1802M)
dam Methylase Kit (New England Biolabs Catalog No. M0222L)
Zymoclean Gel DNA Recovery Kit (Zymo Research Catalog No. D4001)
nprE site-directed mutagenic primers, 100nmole scale, 5' Phosphorylated, PAGE purified
(Integrated DNA Technologies, Inc.)
QUIKCHANGE® Multi Site-Directed Mutagenesis Kit (Stratagene Catalog No. 200514)
MJ Research PTC-200 Peltier Thermal Cycler (Bio-Rad Laboratories)

1.2% agarose E-gels (Invitrogen Catalog No. G5018-01)
TempliPhi Amplification Kit (GE Healthcare Catalog No. 25-6400-10)
Competent *B. subtilis* cells (Δ*aprE*, Δ*nprE*, *oppA*, Δ*spoIIE*, *degUHy32*, Δ*amyE::(xylR,pxylA-comK*)

**Methods:**

To obtain the pUBnprE plasmids containing one mutation (identified through *nprE* SEL screening as described above in Example 3 and in WO 2007/044993, a single colony of each *Bacillus* strain of interest was used to inoculate a 5ml LB + 10 ppm neomycin tube (*e.g.*, starter culture). The culture was grown at 37°C, with shaking at 225 rpm for 6 hours. Then 100 ml of fresh LB + 10ppm neomycin were inoculated with 1ml of the starter culture. This culture was grown overnight at 37°C, with shaking at 225 rpm. Following this incubation, the cell pellet was harvested by sufficient centrifugation to provide a cell pellet. The cell pellet was resuspended in 10 ml Buffer P1 (Qiagen Plasmid Midi Kit). Then, 10μl of Ready-Lyse Lysozyme was added to the resuspended cell pellet and incubated at 37°C for 30 min. The Qiagen Plasmid Midi Kit protocol was continued using 10 ml of Buffer P2 and P3 to account for the increased volume of cell culture. After isolation from *Bacillus* of each pUBnprE plasmid containing a single nprE mutation, the concentration of each plasmid was determined. The plasmids were then *dam* methylated using the *dam* Methylase Kit (New England Biolabs) per the manufacturer's instructions, to methylate approximately 2 μg of each pUBnprE plasmid per tube. The Zymoclean Gel DNA recovery kit was used to purify and concentrate the *dam*-methylated pUBnprE plasmids. The dam-methylated pUBnprE plasmids were then quantitated and diluted to a working concentration of 50 ng/μl for each. Mixed site-directed mutagenic primers were prepared separately for each reaction. For example, using pUBnprE T14R plasmid as the template source, the mixed site-directed mutagenic primer tube would contain 10μl of nprE-S23R, 10 μl nprE-G24R. 10 μl nprE-N46K. and 10 μl nprE-T54R (all primers at 10 μM each). A PCR reaction using the QuikChange Multi Site-Directed Mutagenesis Kit (Stratagene) was performed following the manufacturer's instructions (*e.g.*, 1 μl dam methylated pUBnprE plasmid containing one mutation (50 ng/μl), 2 μl nprE site-directed mutagenic primers (10 μM), 2.5 μl 10x QuikChange Multi Reaction buffer, 1 μl dNTP Mix. 1 μl QuikChange Multi enzyme blend (2.SU/μl), and 17.5 μl distilled, autoclaved water, to provide a 25 μl total reaction mix. The nprE variant libraries were amplified using the following conditions: 95°C. for 1 min. (1st cycle only), followed by 95°C for 1 min, 55°C for 1 min. 65°C for 13.5 min, and repeat cycling 29 times. The reaction product was stored at 4°C overnight. Then, the reaction mixture underwent *Dpn*I digest treatment (supplied with QUIKCHANGE® Multi Site-Directed Mutagenesis Kit) to digest parental pUB-nprE plasmid, using the manufacturer's protocol (*i.e.,* 1.5 μl *Dpn*I restriction enzyme was added to each tube and incubated at 37°C for 3 hours; 2 μl of *Dpn*I-digested PCR reaction was then analyzed on a 1.2% E-gel to ensure PCR reaction worked and that parental template was degraded. TempliPhi rolling circle amplification was then used to generate large amounts of DNA for increasing library size of the nprE multi variants, using the manufacturer's protocol (i.e.. 1 μl Dpnl treated QuikChange Multi Site-Directed Mutagenesis PCR, 5 μl TempliPhi Sample Buffer. 5 μl TempliPhi Reaction Buffer, and 0.2 μl TempliPhi Enzyme Mix, for an ~ 11 μl total reaction; incubated at 30°C for 3 hours; the TempliPhi reaction was diluted by adding 200 μl distilled, autoclaved water and briefly vortexed. Then. 1.5 μl of diluted TempliPhi material was transformed into competent *B. subtilis* cells, and nprE multi variants were selected for using LA + 10 ppm Neomycin + 1.6 % skim milk plates. Colonies were picked and then sequenced to identify the different *nprE* variant library combinations.

Integrated DNA Technologies synthesized all of the primers (100 nmole scale. 5'-phosphorylated, and PAGE purified) used for mutagenesis. Sites evaluated included: 4, 12, 13, 23, 45, 49, 50, 54, 59, 60, 65, 82, 90, 110, 119, 128, 129, 130, 135. 136, 137, 138, 139, 140, 151, 152, 155, 179, 190, 197, 198, 199, 204, 245, 214, 216, 217, 218, 219, 220, 221, 222, 224, 243, 244, 260, 261, 263, 265, 269, 273, 282, 285, 286, 289, 293, 296, 297 and 299. Exemplary mutagenesis primers are described in WO 2007/044993.

## EXAMPLE 4

### Expression, Fermentation, and Purification of Variant Proteases

This Example describes the methods used to express, ferment and purify the proteases of the transformed *B. subtilis* of the preceding examples.

### Neutral Metalloproteases

The recombinant *Bacillus subtilis* was cultivated by conventional batch fermentation in a a nutrient medium. One glycerol vial of a *B. subtilis* culture (containing the *B. amyloliquefaciens* neutral metalloprotease or a variant thereof) was used to inoculate 600 ml of SBG 1% medium containing 200 mg/L chloramphenicol. The cultures were grown for 36-48 hours at 37°C. Alternative methods employed involved the growth of recombinant *B. subtilis* in defined media for 60 hours at 35 "C. The culture fluid was subsequently recovered by centrifugation at 12,000 rpm, as known in the art (SORVALL® centrifuge model RC5B). The secreted neutral metalloproteases were isolated from the culture fluid and concentrated approximately 10-foid using an Amicon filter system 8400 with a BES (polyethersulfone) 10 kDa cutoff.

The concentrated supernatant was dialyzed overnight at 4°C against 25 mM MES buffer, pH 5.4. containing 1 mM CaCl$_2$. The dialyzate was then loaded onto a cation-exchange column Poros HS20 (Applied Biosystems column with a total volume - 83 mL; binding capacity ~ 4.5 g protein/mL column; waters). The column was pre-equilibrated with 25 mM MES buffer, pH 5.4, containing 1 mM CaCl$_2$. The bound protein was eluted using a pH and salt gradient from 25 mM MES, pH 5.4, 1 mM CaCl$_2$ to 50 mM MES, pH 6.2, 2 mM CaCl$_2$ and 100 mM NaCl. Elution of the protein was between pH 5.8 and 6.0. The pure protein was concentrated and buffer-exchanged into 25 mM MES buffer, pH 5.8, containing 2 mM CaCl$_2$, and 40% propylene glycol. The purity of the preparation was assessed by measuring proteolytic activity and by 10 % (w/v) NU-PAGE® Novex SDS-PAGE (Invitrogen Corp.) and found to be greater than 95%.

## EXAMPLE 5

### Identification of the Citrate-Induced Autolytic Sites of NprE Protease

In this Example, methods used to assess the citrate-induced autolysis of wild-type and recombinant variant NprE are described. In these experiments, autolysis of the neutral metalloprotease from *B. amyloliquefaciens* (natural and the recombinant variant expressed in *B. subtilis*) was induced using sodium citrate (Sigma). The autolysis process was controlled by performing the reaction at either: (i) 4°C in 25 mM MES, pH 6.5; or (ii) room temperature in 5 mM HEPES pH 8.0. In these experiments, the autolysis of 0.4 mg/ml NprE was optimized by varying either: (a) the time of incubation (0-120 minutes) in 10 mM citrate; or (b) the citrate concentration (10-100 mM) over 100 minutes. A control of neutral metalloprotease diluted in buffer alone (i.e., no citrate) was incubated under similar conditions. The remaining protease activity was measured using the synthetic peptide (Abz-AGLA-Nba) and calculated relative to its own no-citrate control.

As shown in Figure 1A, the neutral metalloprotease NprE is inactivated in the presence of citrate, a weak calcium chelator. In the presence of 100-250 mM citrate, and in the absence of calcium, the activity of the wild-type neutral protease is decreased to less than 30%, when incubated for 5 minutes at room temperature. This inactivation of protease by citrate is overcome by titration with calcium chloride. Moreover in the presence of 2 mM calcium chloride the wild-type neutral protease is completely stable and enzymatically active. This result demonstrates that depletion of calcium, not zinc, is the cause of NprE instability in the presence of citrate.

The autolytic reactions performed at 4 °C were terminated by addition of an equal volume of 1N HCl. The samples were precipitated using TCA and the pellet was washed and dried using acetone. The resultant pellet was resuspended in 20 μL buffer, pH 6.5, and 4X LDS sample buffer (NuPage, Invitrogen). The autolytic fragments were resolved by 10 % (w/v) SDS-PAGE and electroblotted onto a PVDF membrane. The first 10 amino acid residues were sequenced by Edman degradation (Argo Bioanalytica), and are shown in Table 5-1. The partial amino acid sequences of the autolytic fragments were determined using trypsin in-gel digestion and analyzed using LCQ-MS (Agilent). The in-gel digestion process involved macerating the gel piece that contained the protein, removal of the Coomassie blue stain followed by re-hydration of the gel pieces in 25 mM NH$_4$CO$_3$ containing 2 M urea. Trypsin was added to the re-hydrated gel pieces for approximately 6 hours at 37 °C. Following the digestion, the peptides were extracted using acetonitrile and TCA. The peptides were separated on a C4-hydrophobic column (Vydac) using an acetonitrile- water gradient. The resultant peptide maps were searched with the SEQUEST® database search program against a database containing Genencor enzymes. The amino acid sequences of the first 10 amino acids of each of the fragments were compared with the known amino acid sequence for *B. amyloliquefaciens* NprE. This enabled the identification of the amino acid at the N-termini and hence the cleavage site(s) within NprE.

The autolytic action of citrate on the wild-type neutral protease was studied in the presence of increasing citrate concentrations at 4 °C as shown in Figure 1B. After incubation for 90 minute with 10 mM citrate, two primary autolysis fragments in addition to the remaining intact NprE are observed (lane 1). The molecular weight of intact NprE is approximately 32 kDa, while the primary autolysis fragments are approximately 24 kDa and 9 kDa in size. An increase of the citrate concentration to 100 mM resulted in further autolysis and the generation of additional, secondary autolysis fragments (lanes 4-7). Specifically with increasing citrate, the 24 kDa-autolysis fragment is hydolysed further resulting in three smaller fragments of 21 kDa, 15 kDa and 11kDa.

The N-termini of the fragments were identified by Edman degradation (Figure 2). Fragments 1, 3 and 4 all have the native N-terminal sequence, whereas fragments 2 and 5 have unique N-termini. Fragment 2 is frayed and the start of the N-terminus is at or near D220, A221 or G222. The C-terminus of the 15 kDa fragment was deduced based on its size, and is at or near position L198. In-gel trypsin digestion and LCQ-MS confirmed the identity of these autolysis fragments. Based on the N-terminal and LCQ-MS analysis of the NprE cleavage fragments, primary cleavage sites were identified at amino acid positions M138. L198, D220, A221, and G222.

| Table 5-1. N-Terminal Sequence of NprE Autolytic Fragments | | | |
|---|---|---|---|
| Sample | N-terminal sequence | SEQ ID NO: | MW on SDS-PAGE (kDa) |
| Band 1 | AATTGTGTTL | (SEQ ID NO:8) | 24 |
| Band 2 | DAGDYGGVHT | (SEQ ID NO:9) | 9 |
| | AGDYGGVHTN | (SEQ ID NO:10) | |
| | GDYGGVHTN | (SEQ ID NO:11) | |
| Band 3 | AATTGTGTTL | (SEQ ID NO:8) | 21 |
| Band 4 | AATTGTGTTL | (SEQ ID NO:8) | 15 |
| Band 5 | LSNPTKYGQP | (SEQ ID NO:12) | 11 |

## EXAMPLE 6

**Production of NprE Variants That Are Resistant To Citrate-Induced Autolysis**

This example describes the screening of NprE variants for improved stability in the presence of a calcium chelator. The primary cleavage sites M 138, L198. D220, A221 and G222, as well as amino acids in proximity to these sites and/or on the enzyme surface were targeted for mutagenesis using the gene construction and sequencing methods described in Example 3. and in WO 2007/044993. Site-evaluation libraries for S129. F130, M138. V190 and D220, where the naturally occurring amino acid residue is replaced individually by one of the 19 alternative amino acid residues, were prepared. Likewise, double (M138L-D220P; F130L-D220P, S129I-D220P. V190I-D220P, S129I-V190I, S129V-V190I, S129V-D220P), triple (M 138L-V 190I-D220P, S 129I-F130L-D220P) and quintuple (S 1291-F 130L-M 138L-V 190I-D220P) amino acid substituted variants were constructed.

NprE SEL members were screened in the absence and presence of 50 mM sodium citrate. The buffer used was 25 mM HEPES. pH 8.0. containing 0.1 mM $CaCl_2$. Protease activity was measured using either the succinylated-casein/TNBSA method (Pierce. Inc. Rockford. IL) or the fluorescently labeled Abz-AGLA-Nba peptide assay (Vriend et al.. J Biol Chem. 255:3482-3486, 1980). The spectrophotometer used was the SpectraMax M2[a] (Molecular Devices) and all assays were conducted in medium protein-binding 96-well plates (Corning International). Residual protease activity was calculated for four replicates after incubation at room temperature for 60 minutes. The values were normalized relative to that observed for wild type NprE. The individual variant proteins that showed increased stability towards citrate (relative to wild type) were selected for further characterization. Representative citrate-screemng data employing the DMC/TNBSA endpoint assay for NprE SELs 138 (autolysis site). 190 (surface exposed site) and 220 (autolysis site) are shown in Figure 3.

Similarly the citrate stability of single (S129I/L, F130L, M138I/L, V190I, D220P/E), double (S129I-V190I, S 129V V190I, M138L-D220P, S129I-D220P, F130L-D220P, V 1901-D220P and S129V-D220P), triple (S129I-F130L-D220P and M138L-V190I-D220P) and quintuple (S129I-F130L-M138L-V190I-D220P) NprE variants was assessed by measuring protease activity toward a synthetic peptide and by SDS-PAGE analysis. The citrate-screening data indicated that replacement of M138 by Leu, D220 by Pro or Glu, S 129 by Ile or Leu, F130 by Leu, and V 190 by Leu or Ile resulted in proteases that were less susceptible towards citrate-induced autolysis.

The citrate concentration dependence of stability for select neutral protease variants is shown in Figure 4A. The single amino acid substitution variants, S129I/V, M138I/L and D220E/P, result in 20-30 % greater remaining activity than the wild-type protein, when incubated in the presence of 100 mM citrate at room temperature. V190I shows the greatest remaining activity (~ 60 %) after incubation in 100 mM citrate at room temperature for 60 minutes. A combination of these mutations demonstrates additivity. The most citrate-stable variant is the combination of five individual mutations, S129I-F130L-M138L-V190I-D220P. This quintuple variant S129I-F130L-M138L-V190I-D220P retains all of its activity in the presence of 100 mM citrate for 150 minutes, and the improved stability is substantiated by the absence of autolysis fragments as observed by SDS-PAGE. In particular, the autolysis pattern characteristic of the wild type NprE (lanes 1-4) is contrasted with the absence of autolysis for S129I-F130L-M138L-V190I-D220P (lanes 6-10) in the presence of increasing concentrations of citrate, as shown in Figure 4B. The percentage remaining activity for wild type NprE and variants neutral metalloproteases in the presence of 100 mM citrate after incubation for 60 minutes at room temperature is shown in Figure 6, clearly indicating the additivity of single substitutions to resistance to autolysis.

## EXAMPLE 7

**Differential Scanning Calorimetry (DSC)**

Excessive heat capacity curves were measured using an ultrasensitive scanning high-throughput microcalorimeter. VP-Cap DSC (MicroCal, Inc., Northampton, MA). Approximately 500 □L of 200-to-400 ppm protein was needed. Typically, 400 ppm of NprE and the variant metalloproteases (in the absence and presence 130 mM citrate) were scanned over 20-100 °C temperature range. The same sample was then re-scanned to check the reversibility of the process. For neutral protease the thermal unfolding process was irreversible. The buffer used was 5 mM HEPES, pH 8.0. Scan rate dependence data of the thermal melting points for NprE was assessed over a scan rate of 25 to 200 °C/hr. A scan rate of 200 °C/hr was ultimately selected to minimize any artifacts that may result from aggregation or autolysis. The thermal midpoint (Tm) of the DSC curves was used as an indicator of the thermal stability. 440-ppm wild-type neutral protease displayed a thermal melting point (Tm) of $69.2 \pm 0.5$ °C. The thermal melting points for the wild type and NprE variants is shown in Figure 6 indicating that the mutations have only a minimal effect on the thermal melting point in the absence of citrate.

In contrast, the thermal unfolding of mutant NprE's in the presence of citrate show considerable differences to wild-type NprE (Figure 6). In the presence of 130 mM citrate, pH 8.0, no thermal unfolding profile is obtained for the wild-type protein. This is consistent with its rapid and complete citrate-induced autolysis and inactivation. By comparison, all citrate stable variants showed a thermal-unfolding profile. The thermal unfolding midpoints for wild type NprE and the variants are shown in Figure 5B. In the presence of 130 mM citrate, the least citrate-stable variant characterized using DSC has a thermal melting point of 48 °C, and the most stable single variants have thermal melting points of 52 °C (Figure 6). The citrate-stabilizing substitutions show additivity, and the combination of five point mutations in the protease backbone in the S129IF130L-M138L-V 190I-D220P variant, has a Tm of 59.2 °C. Thus the combination of five improving mutations results in an increase in Tm of +10 °C (Figures 5 and 6). Taken together these results indicate that a correlation exists between resistance to citrate-induced autolysis and thermal stability in the presence of citrate.

## EXAMPLE 8

**Homology Modeling Of NprE**

This example describes the modeling of the structure of *B. amyloliquefaciens* NprE based on the known structure of *B. cereus* NprE, to which it is 45% identical. In addition micro PIXES analysis was used to confirm the stoichiometry and zinc and calcium binding of NprE. This model was used to identify amino acids of NprE that are likely to be solvent exposed.

The co-ordinates for structures used in sequence analysis, alignment and modeling were downloaded from the PDB. The protein sequence of *B. amyloliquefaciens* NprE is found in Swissprot No. P06832, and the sequence of the mature form is set forth as SEQ ID NO:3. The program suite MOE (Chemical Computing Corp Montreal, Canada), was used for alignment calculations and manual re-adjustment, analysis and modeling, according to the manual supplied with the program. The Charmm27 parameter set was chosen for energy minimization calculations.

The structures of zinc neutral proteases from *B. cereus* (1NPC). *B. thermoproteolyticus* (PDB ID 1KEI), *P. aeruginosa* (1 EZM), and S. *aureus* (1BQB) are available. The most closely homologous of these structures to the mature protease domain of *B. amyloliquefaciens* NprE is the *B. cereus* NprE, which was used as the template structure for modeling. They were aligned structurally. Comparison to the sequence alignment revealed a number of insertions and deletions between the *B. amyloliquefaciens* NprE sequence, and the *B. cereus* NprE sequence. Inspection of the alignments revealed likely errors in both, and the alignment was adjusted manually. *B. amyloliquefaciens* NprE has a four residue deletion compared to the template 1NPC structure in a loop in which two $Ca^{2+}$ ions are predicted to bind to the protein. This led to initial difficulty in modeling this very important region. Fortunately, the *S. aureus* NprE, which has the same stoichiometry of metal binding as *B. amyloliquefaciens* NprE, has an identical deletion in this loop. This allowed use of the *S. aureus* NprE structure to guide manual rebuilding in this area, to more accurately model $Ca^{2+}$ binding to the enzyme. Whilst attempting to energy minimize the model, poor results were obtained for the zinc and calcium binding sites. Therefore, the metal ions, and their ligands were kept fixed throughout the energy minimization. Water molecules were not added to the model

The *B. amyloliquefaciens* NprE homology model contains 300 residues of the mature enzyme. A Ramachandron plot of the final model reveals three outliers. They are S23, N61, and S191. Analysis of surface exposed residues shows eight aliphatic or aromatic residues at the surface. They are Y49, L55, I117, F130, V190. L216. V260, and L282. These residues are in surface exposed loops, which are generally those regions least accurately modeled using the homology modeling method, and may indicate regions where the model is less reliable.

The N-terminal part of the *B. amyloliquefaciens* NprE enzyme contains an 8 strand mixed beta sheet, which encloses about 75% of the surface of a long helix. The C-terminal part of the enzyme largely consists of a 6 helical bundle and two short strands of anti-parallel beta-sheet. The active site of the enzyme is found at the interface between these two sub-domains. The substrate binding cleft is very large and open, which is consistent with the observed endo-protease activity.

In the middle of the substrate cleft is found the catalytically important $Zn^{2+}$ ion. The model predicts that H143. H147, and E167 bind this $Zn^{2+}$ ion, with tetrahedral geometry. In thermolysin, the fourth ligand is water, but waters were not added to the *B. amyloliquefaciens* NprE homology model. Residues D139, D178, E180, D181, E186, and D197 form the double calcium site. CA351 of the model is complexed by the side-chains of D139, D178, D181 and E186, and the main-chain carbonyl of D183. CA352 of the model is complexed by the side-chains of D178, E180, D181, and E187. The side-chain of D187 is also near CA352, but it is not clear from the model if it co-ordinates this ion.

To test the prediction of the metal ion stoichiometry of *B. amyloliquefaciens* NprE, micro-Particle-Induced X-ray Emission (micro-PIXE) spectroscopy was performed to empirically determine the zinc and calcium contents. The University of Surrey Ion Bean Centre (Guildford, United Kingdom) was employed for PIXES analysis. As shown in Table 8-1, the observed stoichiometry is 1.02 for $Zn^{2+}$, and 1.62 for $Ca^{2+}$. If partial occupancy is assumed for one or both of the two predicted $Ca^{2+}$ ions, then the analysis is consistent with the prediction. Determination of the binding affinities of $Zn^{2+}$ and $Ca^{2+}$ shows that $Zn^{2+}$ binds 10-fold more tightly than $Ca^{2+}$, consistent with the slight loss of $Ca^{2+}$ observed in the PIXEs sample.

| Table 8-1. Micro-PIXE analysis of *B. amyloliquefaciens* NprE | |
|---|---|
| **Parameter** | **Wild Type NprE** |
| Zn:S ratio | 0.34 |
| Ca:S ratio | 0.54 |
| S content | 3 |
| Zn content per molecule | 1.02 |
| Ca content per molecule | 1.62 |

## EXAMPLE 9

### Assessment Of pH Dependent Activity And Stability

### A. pH dependent activity of wild type NprE and variant NprE:

Different pH buffers were made using a combination of MES monohydrate, Trizma base and sodium acetate (all from Sigma). The final concentration of each component in the buffer was 25mM acetate, 50mM MES and 25mM Tris. The pH of the buffers was adjusted using either sodium hydroxide or hydrochloride to final pH values of 9.40, 8.69, 8.36, 7.70. 7.45, 6.52, 6.00, 5.45, 4.78, and 4.27. AGLA substrate (American Peptide Company) was dissolved in DMF at a concentration of 4.8mM. Different concentrations of AGLA substrate in each buffer were made so that the final AGLA concentrations were 0.096mM. 0.048mM, 0.024mM, 0.012mM, and 0.006mM.

Two hundred $\mu$l of each pH buffer at each substrate concentration was added to one 96 well plate, 10 $\mu$l of 0.94 $\mu$g/ml purified NprE or variant was added to each well, before shaking the plate for 15 sec. The fluorescence from each well was monitored at Excitation 350nm and Emission 415nm using a plate reader (Molecular dynamics) with a reading taken every 11 seconds for 5 mins. The Vmax (maximum linear slope) of each curve was recorded. A plot of Vmax versus AGLA substrate concentration yielded Kcat/Km, which was calculated based on the slope of the curve and the final enzyme concentration used in the substrate. As shown in Figure 8, both wild type NprE and the quintuple NprE variant (S129I-F130L-M138L-V190I-D220P) have an activity optimum towards AGLA substrate of pH 6.5.

### B. pH dependent stability of wild type NprE and variant NprE:

The stability of wild type NprE and the quintuple NprE variant (S129I-F130L-M138L-V190I-D220P = "Quint") at different pH values were tested in the absence and presence of 2mM calcium chloride. The same sets of buffer at different pH were used as described above except 0.005% Tween 80 was also added to each buffer to avoid protein binding to the 96 well plate. To one set of the buffers, 2mM calcium chloride was added. Purified protein was added at a final protein concentration of 10 $\mu$g/ml. The total volume was 200 $\mu$l/well in a 96 well plate. The plate was incubated at room temperature (*e.g.*, 23 °C) for 3 hours. The activity of each protein at varying pH was measured using the AGLA assay. The wild type NprE is most stable at pH 6.5. with decreases in stability observed at pH less than or greater to 6.5. At pH 4.3, NprE has lost 100 % activity. The Quint is very stable in a wide pH range of pH 5.5 to pH 9.4. Although at pH values less than 4.7, stability of Quint decreases. However, the addition of 2mM calcium stabilizes both NprE and the Quint variant at both low pH and high pH.

## Claims

1. An isolated neutral metalloprotease variant having improved stability in the presence of a metal chelator as compared to the stability of the wild-type neutral metalloprotease in the presence of said metal chelator, wherein said variant comprises the mutations M138L/V190I/D220P or S129I/F130L/M138L/V190I/D220P, wherein the numbering of the positions corresponds to the amino acid residues in the amino acid sequence set forth as SEQ ID NO: 3, and wherein said isolated neutral metalloprotease variant has at least about 45% amino identity with the neutral metalloprotease comprising the amino acid sequence set forth as SEQ ID NO: 3.

2. The isolated neutral metalloprotease variant of claim 1, having an improved resistance to citrate-induced autolysis as compared to the resistance of a wild-type neutral metalloprotease.

3. The isolated neutral metalloprotease variant of claim 1, wherein said variant is a *Bacillus* neutral metalloprotease variant, e.g. a *B. amyloliquefaciens* variant.

4. An isolated nucleic acid sequence encoding the neutral metalloprotease variant of any one of claims 1 to 3.

5. An expression vector comprising the nucleic acid sequence of claim 4.

6. A host cell comprising the expression vector of claim 5.

7. The isolated neutral metalloprotease variant of claim 1, wherein said variant comprises an amino acid sequence set forth as SEQ ID NO:19 (M138L/V190I/D220P) or SEQ ID NO:20 (S129I/F130L/M138L/V190I/D220P).

8. A composition comprising the isolated neutral metalloprotease variant of any one of claims 1 to 3.

9. The composition of claim 8, further comprising at least one calcium ion and/or at least one zinc ion, and optionally further comprising citrate.

10. The composition of claim 8, wherein said composition is a cleaning composition, an animal feed, a textile processing composition and/or a leather processing composition.

11. The cleaning composition of claim 10, wherein said composition further comprises at least one additional enzyme or enzyme derivative selected from the group consisting of proteases, amylases, lipases, mannanases, pectinases, cutinases, oxidoreductases, hemicellulases, and cellulases.

12. A cleaning method comprising the step of contacting a surface and/or an article comprising a fabric with the cleaning composition of claim 10, and optionally further comprising the step of rinsing said surface and/or article comprising a fabric.

13. A method for producing a neutral metalloprotease variant according to any one of claims 1 to 3 comprising: transforming a host cell with an expression vector comprising a nucleic acid encoding said neutral metalloprotease variant to produce a transformed host cell; cultivating said transformed host cell under conditions suitable for the production of said neutral metalloprotease variant; producing said neutral metalloprotease variant, and optionally harvesting said produced neutral metalloprotease variant.

14. The method of claim 13, wherein said host cell is a *Bacillus* species, e.g. *B. subtilis.*

## Patentansprüche

1. Isolierte neutrale Metalloprotease-Variante mit verbesserter Stabilität in Gegenwart eines Metallchelators, verglichen mit der Stabilität der neutralen Metalloprotease des Wildtyps in Gegenwart des Metallchelators, wobei die Variante die Mutationen M138L/V190I/D220P oder S129I/F130L/M138L/V190I/D220P umfasst, wobei die Nummerierung der Positionen den Aminosäureresten in der Aminosäuresequenz, angegeben als SEQ ID NO:3, entspricht und wobei die isolierte neutrale Metalloprotease-Variante mindestens etwa 45% Aminosäureidentität mit der neutralen Metalloprotease, umfassend die Aminosäuresequenz, angegeben als SEQ ID NO:3, hat.

2. Isolierte neutrale Metalloprotease-Variante nach Anspruch 1 mit einer verbesserten Beständigkeit gegenüber Citrat-induzierter Autolyse, verglichen mit der Beständigkeit einer neutralen Metalloprotease des Wildtyps.

3. Isolierte neutrale Metalloprotease-Variante nach Anspruch 1, wobei die Variante eine neutrale Metalloprotease-Variante eines *Bacillus,* z.B. eine *B.-amyloliquefaciens*-Variante, ist.

4. Isolierte Nucleinsäuresequenz, codierend die neutrale Metalloprotease-Variante nach einem der Ansprüche 1 bis 3.

5. Expressionsvektor, umfassend die Nucleinsäuresequenz nach Anspruch 4.

6. Wirtszelle, umfassend den Expressionsvektor nach Anspruch 5.

7. Isolierte neutrale Metalloprotease-Variante nach Anspruch 1, wobei die Variante eine Aminosäuresequenz, angegeben als SEQ ID NO:19 (M138L/V190I/D220P) oder SEQ ID NO:20 (S129I/F130L/M138L/V190I/D220P), umfasst.

8. Zusammensetzung, umfassend die isolierte neutrale Metalloprotease-Variante nach einem der Ansprüche 1 bis 3.

9. Zusammensetzung nach Anspruch 8, weiterhin umfassend mindestens ein Calciumion und/oder mindestens ein Zinkion und wahlweise weiterhin umfassend Citrat.

10. Zusammensetzung nach Anspruch 8, wobei die Zusammensetzung eine Reinigungszusammensetzung, ein Tierfutter, eine Textilverarbeitungszusammensetzung und/oder eine Lederverarbeitungszusammensetzung ist.

11. Reinigungszusammensetzung nach Anspruch 10, wobei die Zusammensetzung weiterhin mindestens ein zusätzliches Enzym oder Enzym-Derivat, ausgewählt aus der Gruppe, bestehend aus Proteasen, Amylasen, Lipasen, Mannanasen, Pektinasen, Cutinasen, Oxidoreductasen, Hemicellulasen und Cellulasen, umfasst.

12. Reinigungsverfahren, umfassend den Schritt, eine Oberfläche und/oder einen Gegenstand, umfassend ein Gewebe, mit der Reinigungszusammensetzung nach Anspruch 10 in Kontakt zu bringen, und wahlweise weiterhin umfassend den Schritt, die Oberfläche und/oder den Gegenstand, umfassend ein Gewebe, zu spülen.

13. Verfahren zum Erzeugen einer neutralen Metalloprotease-Variante gemäß einem der Ansprüche 1 bis 3, umfassend: Transformieren einer Wirtszelle mit einem Expressionsvektor, umfassend eine Nucleinsäure, codierend die neutrale Metalloprotease-Variante, um eine transformierte Wirtszelle zu erzeugen; Kultivieren der transformierten Wirtszelle unter Bedingungen, geeignet für die Erzeugung der neutralen Metalloprotease-Variante; Erzeugen der neutralen Metalloprotease-Variante und wahlweise Ernten der erzeugten neutralen Metalloprotease-Variante.

14. Verfahren nach Anspruch 13, wobei die Wirtszelle eine *Bacillus-Spezies,* z.B. *B. subtilis,* ist.

**Revendications**

1. Variant de métalloprotéase neutre isolé possédant une stabilité améliorée en présence d'un chélateur de métal comparée à la stabilité de la métalloprotéase neutre de type sauvage en présence dudit chélateur de métal, où ledit variant comprend les mutations M138L/V190I/D220P ou S129I/F130L/M138L/V190I/D220P, où la numérotation des positions correspond aux résidus d'acide aminé dans la séquence d'acides aminés présentée comme la SEQ ID NO:3 et où ledit variant de métalloprotéase neutre isolé possède au moins environ 45% d'identité d'acides aminés avec la métalloprotéase neutre comprenant la séquence d'acides aminés présentée comme la SEQ ID NO:3.

2. Variant de métalloprotéase neutre isolé selon la revendication 1, possédant une résistance améliorée à une autolyse induite par des citrates comparée à la résistance d'une métalloprotéase neutre de type sauvage.

**3.** Variant de métalloprotéase neutre isolé selon la revendication 1, où ledit variant est un variant de métalloprotéase neutre de *Bacillus,* par ex. un variant de *B. amyloliquefaciens.*

**4.** Séquence d'acide nucléique isolée codant pour le variant de métalloprotéase neutre selon l'une quelconque des revendications 1 à 3.

**5.** Vecteur d'expression comprenant la séquence d'acide nucléique selon la revendication 4.

**6.** Cellule hôte comprenant le vecteur d'expression selon la revendication 5.

**7.** Variant de métalloprotéase neutre isolé selon la revendication 1, où ledit variant comprend une séquence d'acides aminés présentée comme la SEQ ID NO:19 (M138L/V190I/D220P) ou la SEQ ID NO:20 (S129I/F130L/M138L/V190I/D220P).

**8.** Composition comprenant le variant de métalloprotéase neutre isolé selon l'une quelconque des revendications 1 à 3.

**9.** Composition selon la revendication 8, comprenant en outre au moins un ion calcium et/ou au moins un ion zinc et optionnellement comprenant en outre un citrate.

**10.** Composition selon la revendication 8, où ladite composition est une composition nettoyante, une alimentation animale, une composition de traitement de textiles et/ou une composition de traitement du cuir.

**11.** Composition nettoyante selon la revendication 10, où ladite composition comprend en outre au moins une enzyme ou un dérivé d'enzyme supplémentaire choisi dans le groupe constitué de protéases, d'amylases, de lipases, de mannanases, de pectinases, de cutinases, d'oxydoréductases, d'hémicellulases et de cellulases.

**12.** Méthode de nettoyage comprenant l'étape de mise en contact d'une surface et/ou d'un article comprenant un tissu avec la composition nettoyante selon la revendication 10 et optionnellement comprenant en outre l'étape de rinçage de ladite surface et/ou dudit article comprenant un tissu.

**13.** Méthode pour la production d'un variant de métalloprotéase neutre selon l'une quelconque des revendications 1 à 3 comprenant: la transformation d'une cellule hôte avec un vecteur d'expression comprenant un acide nucléique codant pour ledit variant de métalloprotéase neutre pour produire une cellule hôte transformée; la culture de ladite cellule hôte transformée dans des conditions appropriées pour la production dudit variant de métalloprotéase neutre; la production dudit variant de métalloprotéase neutre et optionnellement la récolte dudit variant de métalloprotéase neutre produit.

**14.** Méthode selon la revendication 13, dans laquelle ladite cellule hôte est une espèce de *Bacillus,* par ex. *B. subtilis.*

**FIGURE 1A**

**FIGURE 1B**

**FIGURE 2**

Fragment 1:
1
AATTGTGTTLTVSLNISSESGKYVLRDLSKPTGTQIITYDLQNREYNLPGTLVSSTTNQ
FTTSSQRAAVDAHYNLGKVYDYFYQKFNIVSSVHYGSRSLSNPTKYGQPDNFK

Fragment 2:
220
DAGDYGGVHTAAYNTITKAEQIYYRALTVYLTPSSTFKDAKAALIQSARDLYGSQDAAS
VEAAWNAVGL

Fragment 3:
1
AATTGTGTTLTVSLNISSESGKDLSKPTGTQIITYDLQNREYNLPGTLVSSTTNQFTTS
SQRAAVDAHYNLGKNSYDNKIVSSVHYGSRYNNAAWIGDQMIYGDGDGSFFSPLSGSMD

Fragment 4:
1
AATTGTGTTLTVSLNISSESGKDLSKPTGTQIITYDLQNREYNLPGTLVSSTTNQFTTS
SQRAAVDAHYNLGKNSYDNKIVSSVHYGSRMDV

Fragment 5:
198
LSNPTKYGQPKNYKNLPNTDAGDYGGVHTNSGIPNKAEQIYYRALTVTFKDAKAALIQS
ARDLYGSQDAASVEAAWNAVGL

**FIGURE 3**

FIGURE 4A

**FIGURE 4B**

**FIGURE 5A**

**FIGURE 5B**

**FIGURE 6**

Activity and thermostability of wild type NprE and variants. The percentage remaining activity was determined as described in the Material and Methods using the fluorescence AGLA-assay. The thermal melting point is the midpoint of the DSC unfolding transition.

| Protein | Percentage Remaining activity, 100 mM citrate, 60min | Thermal melting point (oC) | |
|---|---|---|---|
| | | No citrate | 130 mM citrate |
| Wild-type NprE | 0 | 69.7 None | |
| S129I | 35 | 70.7 | 50.3 |
| S129V | 24 | 69.9 | 49.9 |
| F130L | <10 | 69.8 | 48.5 |
| M138I | 37 | 69.2 | 52.5 |
| M138L | 34 | 67.8 | 49.4 |
| V190I | 60 | 69.4 | 50.3 |
| D220E | 25 | 69.8 | 52.2 |
| D220P | 22 | 69.9 | 49.5 |
| S129I V190I | 77 | 70.3 | 55.8 |
| S129V V190I | 78 | 69.9 | 55.3 |
| M138L D220P | 23 | 68.1 | 50.8 |
| S129I D220P | 32 | 70.7 | 51.2 |
| F130L D220P | 35 | 70.3 | 50.8 |
| V190I D220P | 52 | 69.8 | 52.8 |
| S129V D220P | 40 | 70.6 | 55.7 |
| S129I F130L D220P | 50 | 67.5 | 56.6 |
| M138L V190I D220P | 52 | 65.4 | 53.8 |
| S129I F130L M138L V190I D220P | 91 | 68.6 | 59.2 |

## FIGURE 7

SEQ ID NO:18
NprE S129I/F130L/D220P

```
         10         20         30         40         50         60
AATTGTGTTL KGKTVSLNIS SESGKYVLRD LSKPTGTQII TYDLQNREYN LPGTLVSSTT
         70         80         90        100        110        120
NQFTTSSQRA AVDAHYNLGK VYDYFYQKFN RNSYDNKGGK IVSSVHYGSR YNNAAWIGDQ
        130        140        150        160        170        180
MIYGDGDGIL FSPLSGSMDV TAHEMTHGVT QETANLNYEN QPGALNESFS DVFGYFNDTE
        190        200        210        220        230        240
DWDIGEDITV SQPALRSLSN PTKYGQPDNF KNYKNLPNTP AGDYGGVHTN SGIPNKAAYN
        250        260        270        280        290        300
TITKIGVNKA EQIYYRALTV YLTPSSTFKD AKAALIQSAR DLYGSQDAAS VEAAWNAVGL
```

SEQ ID NO:19
NprE M138L/V190I/D220P

```
         10         20         30         40         50         60
AATTGTGTTL KGKTVSLNIS SESGKYVLRD LSKPTGTQII TYDLQNREYN LPGTLVSSTT
         70         80         90        100        110        120
NQFTTSSQRA AVDAHYNLGK VYDYFYQKFN RNSYDNKGGK IVSSVHYGSR YNNAAWIGDQ
        130        140        150        160        170        180
MIYGDGDGSF FSPLSGSLDV TAHEMTHGVT QETANLNYEN QPGALNESFS DVFGYFNDTE
        190        200        210        220        230        240
DWDIGEDITI SQPALRSLSN PTKYGQPDNF KNYKNLPNTP AGDYGGVHTN SGIPNKAAYN
        250        260        270        280        290        300
TITKIGVNKA EQIYYRALTV YLTPSSTFKD AKAALIQSAR DLYGSQDAAS VEAAWNAVGL
```

SEQ ID NO:20
NprE S129I/F130L/M138L/V190I/D220P

```
         10         20         30         40         50         60
AATTGTGTTL KGKTVSLNIS SESGKYVLRD LSKPTGTQII TYDLQNREYN LPGTLVSSTT
         70         80         90        100        110        120
NQFTTSSQRA AVDAHYNLGK VYDYFYQKFN RNSYDNKGGK IVSSVHYGSR YNNAAWIGDQ
        130        140        150        160        170        180
MIYGDGDGIL FSPLSGSLDV TAHEMTHGVT QETANLNYEN QPGALNESFS DVFGYFNDTE
        190        200        210        220        230        240
DWDIGEDITI SQPALRSLSN PTKYGQPDNF KNYKNLPNTP AGDYGGVHTN SGIPNKAAYN
        250        260        270        280        290        300
TITKIGVNKA EQIYYRALTV YLTPSSTFKD AKAALIQSAR DLYGSQDAAS VEAAWNAVGL
```

**FIGURE 8**

**FIGURE 9**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007044993 A
- WO 9934011 A
- US 6376450 B
- US 4683195 A
- US 4683202 A
- US 4965188 A
- US 5322770 A
- US 69925000 A
- US 6605458 B
- WO 9711151 A
- EP 0922499 A
- US 4977252 A
- US 5354559 A
- US 5935826 A
- US 5879584 A
- US 5691297 A
- US 5574005 A
- US 5569645 A
- US 5565422 A
- US 5516448 A
- US 5489392 A
- US 5486303 A
- US 5576282 A
- US 6306812 B1
- US 6326348 B1
- US 4430243 A
- US 5597936 A
- US 5595967 A
- WO 0032601 A
- US 6225464 B
- US 4515705 A
- US 4537706 A
- US 4515707 A
- US 4550862 A
- US 4561998 A
- US 4597898 A
- US 4968451 A
- US 5565145 A
- US 5929022 A
- US 6294514 B
- US 63764451 B
- WO 2002014490 A
- WO 0214490 A

**Non-patent literature cited in the description**

- **GALANTE.** *Current Organic Chemistry.,* 2003, vol. 7, 1399-1422
- **SHOWELL.** Handbook of Detergents, Part D: Formulation. Taylor and Francis, 2006
- **EIJSINK et al.** *J Biotechnol,* 2004, vol. 113, 105-120
- **EIJSINK et al.** *Nat Struct Biol,* 1995, vol. 2, 374-379
- **VAN DEN BURG et al.** *Biotechnol Appl Bioeng,* 1999, vol. 30, 35-40
- **VRIEND. J.** *Comput Aided Mol Des.,* 1993, vol. 7, 367-396
- **SAMBROOK et al.** Molecular Cloning: A Laboratory Manual. Cold Spring Harbor, 1989
- **AUSUBEL et al.** Current Protocols in Molecular Biology. 1987
- **SINGLETON ; SAINSBURY.** Dictionary of Microbiology and Molecular Biology. John Wiley and Sons, 1994
- **HALE ; MARHAM.** The Harper Collins Dictionary of Biology. Harper Perennial, 1991
- Microbial Proteinases. **KALISZ.** Advances in Biochemical Engineering/Biotechnology. 1988
- **DEL MAR et al.** *Anal Biochem,* 1979, vol. 99, 316-320
- Genetics. **FERRARI et al.** Bacillus. Plenum Publishing Corp, 1989, 57-72
- **GUEROT-FLEURY.** *Gene,* 1995, vol. 167, 335-337
- **PALMEROS et al.** *Gene,* 2000, vol. 247, 255-264
- **TRIEU-CUOT et al.** *Gene,* 1983, vol. 23, 331-341
- **SMITH ; WATERMAN.** *Adv Appl Math,* 1981, vol. 2, 482
- **NEEDLEMAN ; WUNSCH.** *J Mol Biol,* 1970, vol. 48, 443
- **PEARSON ; LIPMAN.** *Proc Natl Acad Sci USA,* 1988, vol. 85, 2444
- **DEVEREUX et al.** *Nucl Acid Res,* 1984, vol. 12, 387-395
- **FENG ; DOOLITTLE.** *J Mol Evol,* 1987, vol. 35, 351-360
- **HIGGINS ; SHARP.** *CABIOS,* 1989, vol. 5, 151-153

- **ALTSCHUL et al.** *J Mol Biol,* 1990, vol. 215, 403-410
- **KARLIN et al.** *Proc Natl Acad Sci USA,* 1993, vol. 90, 5873-5787
- **ALTSCHUL et al.** *Meth Enzymol,* 1996, vol. 266, 460-480
- **KACIAN et al.** *Proc Natl Acad Sci USA,* 1972, vol. 69, 3038
- **CHAMBERLIN et al.** *Nature,* 1970, vol. 228, 227
- **WU ; WALLACE.** *Genomics,* 1989, vol. 4, 560
- **DYNAN ; TIJAN.** *Nature,* 1985, vol. 316, 774-78
- **VRIEND et al.** Stability and Stabilization of enzymes. Elsevier, 1993, 93-99
- Biomedical and Health Research. **VAN DEN BURG et al.** Proteolysis in Cell Functions Manipulating the Autolytic Pathway of a Bacillus Protease. IOS Press, 1997, vol. 13, 576
- **DÜRRSCHMIDT et al.** *FEBS J.,* 2005, vol. 272, 1523-1534
- **GALANTE ; FORMANTICI.** *Curr. Organic Chem..,* 2003, vol. 7, 1399-1422
- **GRYCZAN.** *J Bacteriol,* 1978, vol. 134, 318-329
- **NEIDHARDT et al.** *J Bacteriol.,* 1974, vol. 119, 736-747
- **VRIEND et al.** *J Biol Chem.,* 1980, vol. 255, 3482-3486